(19) Europäisches Patentamt
European Patent Office
Office européen des brevets

(11) EP 1 171 444 B1

(12) **FASCICULE DE BREVET EUROPEEN**

(45) Date de publication et mention de la délivrance du brevet:
**02.07.2003 Bulletin 2003/27**

(21) Numéro de dépôt: **00917149.7**

(22) Date de dépôt: **07.04.2000**

(51) Int Cl.⁷: **C07D 495/14**, A61K 31/55, A61P 5/00 // (C07D495/14, 333:00, 243:00), C07D221:00

(86) Numéro de dépôt international:
**PCT/FR00/00881**

(87) Numéro de publication internationale:
**WO 00/061587 (19.10.2000 Gazette 2000/42)**

(54) **PYRIDO-THIENO-DIAZEPINES, LEUR PROCEDE DE PREPARATION ET LES COMPOSITIONS PHARMACEUTIQUES LES CONTENANT**

PYRIDOTHIENODIAZEPINE, VERFAHREN ZU IHRER HERSTELLUNG UND DIESE ENTHALTENDE PHARMAZEUTISCHE ZUBEREITUNGEN

PYRIDO-THIENO-DIAZEPINES, METHOD FOR THE PRODUCTION THEREOF AND PHARMACEUTICAL COMPOSITIONS CONTAINING SAID PYRIDO-THIENO-DIAZEPINES

(84) Etats contractants désignés:
**AT BE CH CY DE DK ES FI FR GB GR IE IT LI LU MC NL PT SE**

(30) Priorité: **09.04.1999 FR 9904440**

(43) Date de publication de la demande:
**16.01.2002 Bulletin 2002/03**

(73) Titulaire: **SOCIETE DE CONSEILS DE RECHERCHES ET D'APPLICATIONS SCIENTIFIQUES (S.C.R.A.S.)**
**75016 Paris (FR)**

(72) Inventeurs:
- **Liberatore, Anne-Marie**
  **Saint Benoist, 78610 Auffargis (FR)**
- **Bigg, Dennis**
  **F-91190 Gif-sur-Yvette (FR)**

(74) Mandataire: **Bourgouin, André**
**Beaufour Ipsen - S.C.R.A.S.,**
**24, rue Erlanger**
**75781 Paris Cedex 16 (FR)**

(56) Documents cités:
**GB-A- 2 242 427** **US-A- 4 623 646**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 171 444 B1

Printed by Jouve, 75001 PARIS (FR)

## Description

La présente invention concerne de nouvelles pyrido-thiéno-diazépines, leur procédé de préparation et les compositions pharmaceutiques les contenant. Ces diazépines sont particulièrement intéressantes pour traiter les états pathologiques ou les maladies dans lesquels un (ou plusieurs) des récepteurs de la somatostatine est (sont) impliqué (s).

La somatostatine (SST) a été isolée pour la première fois en tant que facteur inhibant la sécrétion de l'hormone de croissance (Brazeau P. et al., *Science* 1973, 179, 77-79). Cette substance est connue sous deux formes somatostatine 14 et somatostatine 28 et est largement distribuée dans le règne animal et chez l'homme. Les peptides de cette famille interviennent également en tant que neurotransmetteurs dans le cerveau (hypothalamus, neurones sensitifs, cortex cérébral) (Reisine T. et al., *Neuroscience* 1995, 67, 777-790 ; Reisine et al., *Endocrinology* 1995, 16:427-442) et dans les organes endocriniens (pancréas, intestin, reins, glandes salivaires, cellules C thyroïdiennes...). La bioactivité de la somatostatine dépend directement d'une famille de cinq récepteurs récemment clonés.

Parmi les désordres pathologiques associés à la somatostatine (Moreau J.P. et al., Life Sciences, 1987, 40, 419 ; Harris A.G. et al., The European Journal of Medicine, 1993, 2, 97-105), on peut citer par exemple: l'acromégalie, les adénomes hypophysaires ne sécrétant pas d'hormones de croissance, les adénomes hypophysaires sécrétant de la thyréostimuline, la maladie de Cushing, les gonadotrophinomes et les prolactinomes, les effets secondaires cataboliques des glucocorticoïdes, les adénomes hypophysaires sans action endocrinienne, le diabète insulinodépendant, la rétinopathie diabétique, la néphropathie diabétique, l'hyperthyroïdie, le gigantisme, les tumeurs gastroentéropancréatiques endocriniennes dont le syndrome carcinoïde, le VIPome, l'insulinome, la nésidioblastose, l'hyperinsulinémie, le glucagonome, le gastrinome et le syndrome de Zollinger-Ellison, le GRFome ainsi que le saignement aigu des varices oesophagiennes, le reflux gastrooesophagien, le reflux gastroduodénal, la pancréatite, les fistules entérocutanées et pancréatiques mais aussi les diarrhées, les diarrhées réfractaires du syndrome d'immunodépression acquise, la diarrhée chronique sécrétoire, la diarrhée associée avec le syndrome de l'intestin irrité, troubles liés au peptide libérateur de gastrine, les pathologies secondaires aux greffes intestinales, l'hypertension portale ainsi que les hémorragies des varices chez des malades avec cirrhose, l'hémorragie gastro-intestinale, l'hémorragie de l'ulcère gastroduodénale, la maladie de Crohn, les scléroses systémiques, le dumping syndrome, le syndrome du petit intestin, l'hypotension, la sclérodermie et le carcinome thyroïdien médullaire, les maladies liées à l'hyperprolifération cellulaire comme les cancers et plus particulièrement le cancer du sein, le cancer de la prostate, le cancer thyroïdien ainsi que le cancer pancréatique et le cancer colorectal, les fibroses et plus particulièrement la fibrose du rein, la fibrose du foie, la fibrose du poumon, la fibrose de la peau, également la fibrose du système nerveux central ainsi que celle du nez et la fibrose induite par la chimiothérapie, et d'autres domaines thérapeutiques comme, par exemple, les céphalées y compris les céphalées associées aux tumeurs hypophysaires, les douleurs, les accès de panique, la chimiothérapie, la cicatrisation des plaies, l'insuffisance rénale résultant d'un retard de croissance, l'obésité et retard de croissance lié à l'obésité, le retard de croissance utérin, la dysplasie du squelette, le syndrome de Noonan, le syndrome d'apnée du sommeil, la maladie de Graves, la maladie polykystique des ovaires, les pseudo kystes pancréatiques et ascites, la leucémie, le méningiome, la cachexie cancéreuse, l'inhibition des H pylori, le psoriasis, l'ostéoporose ainsi que la maladie d'Alzheimer.

Ces diazépines présentent une affinité et une sélectivité pour les récepteurs de la somatostatine. Les applications cliniques de la somatostatine naturelle et de ses analogues peptidiques sont souvent limitées. En effet, une mauvaise biodisponibilité par voie orale et une faible sélectivité en sont souvent la cause principale (Robinson, C., Drugs of the Future, 1994, 19, 992 ; Reubi, J.C. et al., TIPS, 1995, 16, 110). En raison de leur structure non peptidique, les composés de la présente invention, agonistes ou antagonistes de la somatostatine, semblent moins susceptibles de dégradation métabolique que l'hormone naturelle et ses analogues peptidiques et aurait ainsi une durée d'action supérieure. Ces composés peuvent être avantageusement utilisés pour traiter les états pathologiques ou les maladies tels que présentés ci-dessus et dans lesquels un (ou plusieurs) des récepteurs de la somatostatine est(sont) impliqué (s).

La présente invention a ainsi pour objet les composés de formule générale I

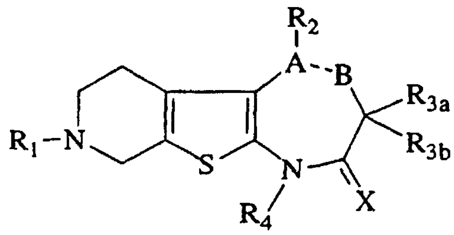

I

sous forme racémique, ou d'énantiomères ou de diastéréoisomères ou toutes combinaisons de ces formes, et dans laquelle

$R_1$ représente l'atome d'hydrogène ou un radical de formule $R'_1$-NH-C(Y)- ;

$R'_1$ représente un radical aryle ou hétéroaryle, les radicaux aryle et hétéroaryle étant éventuellement substitués ;

$R_2$ représente un radical alkyle inférieur, trifluorométhyle ou le radical phényle éventuellement substitué ;

X et Y représentent indépendamment O ou S ;

$R_{3a}$ représente l'atome d'hydrogène, un radical alkyle inférieur, hydroxy ou le radical de formule -OC(O) $R'_{3a}$ ;

$R'_{3a}$ représente un radical alkyle comprenant de 1 à 10 atomes de carbone éventuellement substitué ;

$R_{3b}$ représente l'atome d'hydrogène ou un radical alkyle inférieur ;

$R_4$ représente un radical de formule $-(CH_2)_n-CHR'_4R''_4$ ;

n représente les valeurs 0, 1, 2, 3, 4, 5 ou 6 ;

$R'_4$ et $R''_4$ représentent, indépendamment, l'atome d'hydrogène, un radical alkyle inférieur, cycloalkyle, cycloalkyle alkyle inférieur, aryle, arylalkyle inférieur, hétéroaryle, hétéroarylalkyle inférieur, arylcarbonyle ou adamantyle, ces radicaux étant éventuellement substitués ;

A---B représente -C=N- ou $-C-N(R_5)-$ ;

$R_5$ représente l'atome d'hydrogène, un radical alkyle inférieur, alkenyle inférieur ou de formule $-C(O)-(CH_2)_p-R'_5$ ;

$R'_5$ représente l'atome d'hydrogène, un radical amino, alkyl inférieur amino, di(alkyl inférieur)amino, cycloalkyle, hétérocycloalkyle, guanidyle éventuellement substitué par nitro ou cyano, aryle éventuellement substitué, hétéroaryle ou un radical de formule $-NH-C(O)-(CH_2)_c-NH-C(O)-(CH_2)_d-NH_2$ ;

p représente les valeurs 0, 1, 2, 3, 4, 5, 6, 7, 8, 9 ou 10 ;

c et d représentent indépendamment les valeurs 0, 1, 2 ou 3 ;

ou un sel de ces composés, dans lesquels

le ou les substituants, identiques ou différents, que peuvent porter les radicaux aryle et hétéroaryle que représente $R'_1$, sont choisis parmi les radicaux suivants : alkyle inférieur, alkoxy inférieur, alkylthio inférieur, alkoxy inférieur carbonyle, alkyle inférieur sulfonyle, halo, trifluorométhyle, trifluorométhyloxy, hydroxy, nitro, cyano, aryle, aryloxy, cycloalkyle ou hétérocycloalkyle ;

le ou les substituants, identiques ou différents, que peut porter le radical phényle que représente $R_2$, sont choisis parmi : le radical hydroxy, halo, un radical alkyle inférieur ou alkoxy inférieur ;

le ou les substituants, identiques ou différents, que peut porter le radical alkyle que représente $R'_{3a}$, sont choisis parmi les radicaux suivants : cycloalkyle ; hétérocycloalkyle ; aryle ; hétéroaryle ; guanidyle éventuellement substitué par nitro ou cyano ; un radical de formule $NR''_{3a}R'''_{3a}$ dans laquelle $R''_{3a}$ et $R'''_{3a}$ représentent, indépendamment, l'atome d'hydrogène, un radical alkyle inférieur, aryle, arylalkyle inférieur, hétéroarylalkyle inférieur, alkylcarbonyle ou alkoxycarbonyle ;

le ou les susbtituants que peuvent porter les radicaux alkyle inférieur, cycloalkyle, cycloalkyle alkyle inférieur, aryle, arylalkyle inférieur, hétéroaryle, hétéroarylalkyle inférieur, arylcarbonyle ou adamantyle que représentent $R'_4$ et $R''_4$, sont choisis parmi : le radical hydroxy, halo, trifluorométhyle, un radical alkyle inférieur ou alkoxy inférieur ;

le ou les substituants, identiques ou différents, que peut porter le radical aryle que réprésente $R'_5$ sont choisis parmi les radicaux alkyle et alkoxyalkyle, les radicaux alkyle et alkoxyalkyle étant eux même éventuellement substitués par les radicaux oxy et amino.

Dans les définitions indiquées ci-dessus, l'expression halo représente le radical fluoro, chloro, bromo ou iodo, de préférence chloro, fluoro ou bromo. L'expression alkyle inférieur représente un radical alkyle ayant de 1 à 6 atomes de carbone, linéaire ou ramifié, et en particulier un radical alkyle ayant de 1 à 4 atomes de carbone tels que les radicaux méthyle, éthyle, propyle, isopropyle, butyle, isobutyle, sec-butyle et tert-butyle, mais peut également représenter un radical pentyle, néopentyle, hexyle ou isohexyle.

Les radicaux alkoxy inférieurs peuvent correspondre aux radicaux alkyles indiqués ci-dessus comme par exemple les radicaux méthoxy, éthoxy, propyloxy ou isopropyloxy mais également butoxy linéaire, secondaire ou tertiaire. Le terme alkylthio inférieur désigne de préférence les radicaux dans lesquels le radical alkyle est tel que défini ci-dessus comme par exemple méthylthio, éthylthio. Le terme alkenyle inférieur désigne de préférence le radical alkenyle ayant de 1 à 6 atomes de carbones comme par exemple vinyle, allyle, butényle. Le terme alkoxyalkyle désigne le radical dans lequel les radicaux alkoxy et alkyle sont tels que définis ci-dessus.

Le terme cycloalkyle désigne de préférence les cycles cyclopropyle, cyclobutyle, cyclopentyle ou cyclohexyle. L'expression hétérocycloalkyle désigne un cycloalkyle saturé contenant de 2 à 7 atomes de carbones et au moins un hétéroatome. Ce radical peut contenir plusieurs hétéroatomes identiques ou différents. De préférence, les hétéroatomes sont choisis parmi l'oxygène, le soufre ou l'azote. Comme exemples d'hétérocycloalkyle, on peut citer le cycle pyrrolidine, imidazolidine, pyrrazolidine, isothiazolidine, thiazolidine, isoxazolidine, oxazolidine, pipéridine, pipérazine ou morpholine.

L'expression aryle représente un radical aromatique, constitué d'un cycle ou de cycles condensés, comme par exemple le radical phényle ou naphtyle. Le terme aryloxy désigne de préférérence les radicaux dans lesquels le radical aryle est tel que défini ci-dessus comme par exemple le radical phénoxy. L'expression hétéroaryle désigne un radical aromatique, constitué d'un cycle ou de cycles condensés, avec au moins un cycle contenant un ou plusieurs hétéroatomes identiques ou différents choisis parmi le soufre, l'azote ou l'oxygène. Comme exemple de radical hétéroaryle, on peut citer les radicaux thiényle, furyle, pyrrolyle, imidazolyle, pyrazolyle, isothiazolyle, thiazolyle, isoxazolyle, oxazolyle, pyridyle, pyrazinyle, pyrimidyle, benzothiényle, benzofuryle et indolyle.

Les radicaux arylalkyles inférieurs désignent les radicaux dans lesquels respectivement les radicaux aryle et alkyle inférieur sont tels que définis ci-dessus comme par exemple benzyle, phenéthyle ou naphtylméthyle. Les radicaux hétéroarylalkyles inférieurs désignent les radicaux dans lesquels respectivement les radicaux hétéroaryle et alkyle inférieur sont tels que définis ci-dessus comme par exemple indolylméthyle, thiénylméthyle, furylméthyle. Le terme cycloalkyl alkyle inférieur désignent les radicaux dans lesquels respectivement les radicaux cycloalkyl et alkyle inférieur sont tels que définis ci-dessus.

Le terme alkylsulfonyle désigne de préférence les radicaux dans lesquels le radical alkyle est tel que défini ci-dessus. De même, les termes arylcarbonyle, alkoxycarbonyle et alkylcarbonyle désignent de préférence les radicaux dans lesquels les radicaux aryle, alkoxy et alkyl sont tels que définis ci-dessus. Les termes alkyl inférieur amino et dialkyl inférieur amino désignent de préférence les radicaux dans lesquels les radicaux alkyles sont tels que définis ci-dessus, comme par exemple méthylamino, éthylamino, diméthylamino, diéthylamino ou (méthyl)(éthyl)amino.

Selon la définition des groupes variables, un composé de formule I tel que défini ci-dessus peut présenter un ou plusieurs carbones asymétriques. L'invention concerne les composés de formule I telle que définie ci-dessus, composés qui peuvent se trouver sous forme racémique, ou d'énantiomères ou diastéréoisomères ou toutes combinaisons de ces formes.

L'invention a plus particulièrement pour objet des composés de formule générale I telle que définie ci-dessus, dans laquelle

$R'_1$ représente un radical aryle éventuellement substitué par un ou plusieurs substituants, identiques ou différents, choisis parmi les radicaux suivants : alkoxy inférieur, trifluorométhyle ou nitro ;

$R_2$ représente un radical alkyle inférieur ou le radical phényle éventuellement substitué par un ou plusieurs groupes, identiques ou différents, choisis parmi : halo ou alkyle inférieur ;

$R_{3a}$ représente l'atome d'hydrogène, hydroxy ou le radical de formule -OC(O) $R'_{3a}$ ;

$R'_{3a}$ représente un alkyle, linéaire ou ramifié, comprenant de 1 à 6 atomes de carbone éventuellement substitué par un ou plusieurs substituants, identiques ou différents, de formule $NR''_{3a}R'''_{3a}$ dans laquelle $R''_{3a}$ et $R'''_{3a}$ représentent, indépendamment, l'atome d'hydrogène, un radical alkyle inférieur ou alkoxycarbonyle.

$R_{3b}$ représente l'atome d'hydrogène ;

$R'_4$ et $R''_4$ représentent, indépendamment, l'atome d'hydrogène, un radical alkyle inférieur, cycloalkyle, aryle, hétéroaryle, arylcarbonyle ou adamantyle ;

A---B représente -C=N,

et de manière préférentielle,

$R'_1$ représente un radical phényle éventuellement substitué par un ou plusieurs substituants, identiques ou différents, choisis parmi les radicaux suivants : alkoxy inférieur, trifluorométhyle ou nitro ;

$R_2$ représente un radical alkyle inférieur ou le radical phényl éventuellement substitué par un ou plusieurs groupes, identiques ou différents, choisis parmi : alkyle inférieur, chloro ou fluoro ;

$R'_{3a}$ représente un alkyle, linéaire ou ramifié, comprenant de 1 à 6 atomes de carbone éventuellement substitué par un ou plusieurs groupes amine ;

$R'_4$ et $R''_4$ représentent, indépendamment, l'atome d'hydrogène, alkyle inférieur, cyclohexyle, phényle, pyridyle, phénylcarbonyle ou adamantyle.

Plus particulièrement, l'invention a pour objet les composés décrits ci-après dans les exemples et dans lesquels A---B représente -C=N-, en particulier les composés dans lesquels les radicaux $R_1$ ; $R'_1$ ; Y ; $R_2$ ; $R_{3a}$ ; $R_{3b}$ ; X ; n ; $R'_4$ ; $R''_4$ ont respectivement les significations suivantes :

H ; - ; - : 2-Cl-Phe ; H ; H ; O ; 1 ; H ; Phe ;

H ; - ; - ; 2-Cl-Phe ; H ; H ; S ; 1 ; H ; Phe ;

H ; - ; - ; 2-Cl-Phe ; H ; H ; O ; 2 ; H ; Phe ;

H ; - ; - ; 2-Cl-Phe ; H ; H ; O ; 0 ; H ; phénylcarbonyle ;

H ; - ; - ; 2-Cl-Phe ; H ; H ; O ; 0 ; H ; Phe ;

H ; - ; - ; 2-Cl-Phe ; H ; H ; O ; 0 ; H ; cyclohexyle ;

H; -; - ; 2-Cl-Phe ; H ; H ; O ; 4 ; H ; H ;

H ; - ; - ; 2-Cl-Phe ; H ; H ; O ; 2 ; Phe ; Phe ;

H ; - ; - ; 2-Cl-Phe ; H ; H ; O ; 2 ; Me ; Me ;

H ; - ; - ; 2-Cl-Phe ; H ; H ; O ; 0 ; H ; adamantyle ;

H ;- ; - ; 2-Cl-Phe ; H ; H ; O ; 1 ; H ; pyridyle ;

H ; - ; - ; Phe ; H ; H ; O ; 1 ; H ; Phe ;

H ; - ; - ; 4-Cl-Phe ; H ; H ; O ; 1 ; H ; Phe ;

H ; - ; - ; 2-F-Phe ; H ; H ; O ; 1 ; H ; Phe ;

H ; - ; - ; 4-F-Phe ; H ; H ; O ; 1 ; H ; Phe ;

H ; - ; - ; 2-Me-Phe ; H ; H ; O ; 1 ; H ; Phe ;

H ; - ; - ; t-butyle ; H ; H ; O ; 1;H ; Phe ;

H ; - ; - ; 2-Cl-Phe ; OH ; H ; O ; 1 ; H ; Phe ;

H ; - ; - ; 2-Cl-Phe ; OC(O)-$(CH_2)_6NH_2$ ; H ; O ; 1 ; H ; Phe ;

$R'_1$-NR-C(Y)- ; 2-$NO_2$-4-MeO-Phe ; S ; 2-Cl-Phe ; H ; H ; O ; 1 ; H ; Phe ;

$R'_1$-NH-C(Y)- ; 2-$NO_2$-4-MeO-Phe ; S ; 2-Cl-Phe ; H ; H ; O ; 2 ; H ; Phe ;

$R'_1$-NH-C(Y)- ; 2-$NO_2$-4-MeO-Phe ; S ; 2-Cl-Phe ; H ; H ; S ; 1 ; H ; Phe ;

$R'_1$-NH-C(Y)- ; 2-$NO_2$-4-MeO-Phe ; S ; 2-Cl-Phe ; H ; H ; O ; 0 ; H ; phénylcarbonyle ;

$R'_1$-NH-C(Y)- ; 2-$NO_2$-4-MeO-Phe ; S ; 2-Cl-Phe ; H ; H ; O ; 0 ; H ; Phe ;

$R'_1$-NH-C(Y)- ; 2-$NO_2$-4-MeO-Phe ; S ; 2-Cl-Phe ; H ; H ; O ; 4 ; H ; H ;

$R'_1$-NH-C(Y)- ; 2-$NO_2$-4-MeO-Phe ; S ; 2-Cl-Phe ; H ; H ; O ; 0 ; H ; cyclohexyle ;

$R'_1$-NH-C(Y)- ; 2-$NO_2$-4-MeO-Phe ; S ; 2-Cl-Phe ; H ; H ; O ; 2 ; Phe ; Phe ;

$R'_1$-NH-C(Y)- ; 2-$NO_2$-4-MeO-Phe ; S ; 2-Cl-Phe ; H ; H ; O ; 2 ; Me ; Me ;

$R'_1$-NH-C(Y)- ; 2-$NO_2$-4-MeO-Phe ; S ; 2-Cl-Phe ; H ; H ; O ; 0 ; H ; adamantyle ;

$R'_1$-NH-C(Y)- ; 2-$NO_2$-4-MeO-Phe ; S ; 2-Cl-Phe ; H ; H ; O ; 1 ; H ; pyridyle ;

$R'_1$-NH-C(Y)- ; 2-$NO_2$-4-MeO-Phe ; S ; Phe ; H ; H ; O ; 1 ; H ; Phe ;

$R'_1$-NH-C(Y)- ; 2-$NO_2$-4-MeO-Phe ; S ; 4-Cl-Phe ; H ; H ; O ; 1 ; H ; Phe ;

$R'_1$-NH-C(Y)- ; 2-$NO_2$-4-MeO-Phe ; S ; 2-F-Phe ; H ; H ; O ; 1 ; H ; Phe ;

$R'_1$-NH-C(Y)- ; 2-$NO_2$-4-MeO-Phe ; S ; 4-F-Phe ; H ; H ; O ; 1 ; H ; Phe ;

$R'_1$-NH-C(Y)- ; 2-$F_3C$-Phe ; O ; 4-F-Phe ; H ; H ; O ; 1 ; H ; Phe ;

$R'_1$-NH-C(Y)- ; 2-$NO_2$-4-MeO-Phe ; S ; 2-Me-Phe ; H ; H ; O ; 1 ; H ; Phe ;

$R'_1$-NH-C(Y)- ; 2-$NO_2$-4-MeO-Phe ; S ; t-butyle ; H ; H ; O ; 1 ; H ; Phe ;

$R'_1$-NH-C(Y)- ; 2-$NO_2$-4-MeO-Phe ; S ; 2-Cl-Phe ; OH ; H ; O ; 1 ; H ; Phe ;

$R'_1$-NH-C(Y)- ; 2-$NO_2$-4-MeO-Phe ; S ; 2-Cl-Phe ; OC(O)-$(CH_2)_6NH_2$ ; H ; O ; 1 ; H ; Phe.

L'invention a plus particulièrement pour objet également des composés de formule générale I telle que définie ci-dessus dans laquelle

$R'_1$ représente un radical aryle éventuellement substitué par un ou plusieurs substituants, identiques ou différents,

choisis parmi les radicaux suivants : alkoxy inférieur ou nitro ;

$R_2$ représente un radical alkyle inférieur ou phényle éventuellement substitué par un ou plusieurs groupes halo identiques ou différents ;

$R_{3a}$ et $R_{3b}$ représente l'atome d'hydrogène ;

$R'_4$ et $R''_4$ représentent, indépendamment, l'atome d'hydrogène, un radical alkyle inférieur ou aryle ;

A---B représente $-C-N(R_5)-$ ;

$R_5$ représente l'atome d'hydrogène, un radical alkenyle inférieur ou de formule $-C(O)-(CH_2)_p-R'_5$ ;

et de manière préférentielle,

$R'_1$ représente un radical phényle éventuellement substitué par un ou plusieurs substituants, identiques ou différents, choisis parmi les radicaux suivants : alkoxy inférieur ou nitro ;

$R_2$ représente un radical alkyle inférieur ou phényle éventuellement substitué par un groupe chloro ;

$R'_4$ et $R''_4$ représentent, indépendamment, l'atome d'hydrogène, alkyle inférieur ou phényle ;

$R_5$ représente l'atome d'hydrogène, pentenyle ou un radical de formule $-C(O)-(CH_2)_p-R'_5$ ;

$R'_5$ représente l'atome d'hydrogène, un radical amino, cyclopentyle, indolyle, de formule $-NH-C(O)-CH_2-NH-C(O)-CH_2-NH_2$, ou phényle éventuellement substitué par un ou plusieurs substituants, identiques ou différents, choisis parmi les radicaux alkyle et alkoxyalkyle, les radicaux alkyle et alkoxyalkyle étant eux même substitués par les radicaux oxy et amino.

Plus particulièrement, l'invention a pour objet les composés décrits ci-après dans les exemples et dans lesquels A---B représente $-C-N(R_5)-$, en particulier les composés dans lesquels les radicaux $R_1$ ; $R'_1$ ; Y ; $R_2$ ; $R_{3a}$ ; $R_{3b}$ ; $R_5$ ; X ; n ; $R'_4$ ; $R''_4$ ont respectivement les significations suivantes :

H ; - ; - ; 2-Cl-Phe ; H ; H ; H ; O ; 1 ; H ; Phe ;

H ; - ; - ; 2-Cl-Phe ; H ; H ; H ; O ; 2 ; Me ; Me ;

H ; - ; - ; 2-Cl-Phe ; H ; H ; $-CH_2CH=C(Me)_2$ ; O ; 1 ; H ; Phe ;

H ; - ; - ; 2-Cl-Phe ; H ; H ; aminohexylcarbonyl ; O ; 1 ; H ; Phe ;

H ;- ; - ; 2-Cl-Phe ; H ; H ; aminopentylcarbonyl ; O ; 1 ; H ; Phe ;

H ; - ; - ; 2-Cl-Phe ; H ; H ; indolylméthylcarbonyl ; O ; 1 ; H ; Phe ;

H ; - ; - ; 2-Cl-Phe ; H ; H ; aminobutylcarbonyl ; O : 1 : H ; Phe ;

H ; - ; - ; 2-Cl-Phe ; H ; H ; propylcarbonyl ; O ; 1 ; H ; Phe ;

H ; - ; - ; 2-Cl-Phe ; H ; H ; cyclopentyl-méthylcarbonyl ; O ; 1 ; H ; Phe ;

H ; - ; - ; 2-Cl-Phe ; H ; H ; phényl-propylcarbonyl ; O ; 1 ; H ; Phe ;

H ; - ; - ; 2-Cl-Phe ; H ; H ; phényléthylcarbonyl ; O ; 1 ; H ; Phe ;

H ; - ; - ; 2-Cl-Phe ; H ; H ; 4-(L-alanoyloxyméthyl)benzyl carbonyl ; O ; 1 ; H ; Phe ;

H ; - ; - ; 2-Cl-Phe ; H ; H ; 4-aminométhyl-phénylcarbonyl ; O ; 1 ; H ; Phe ;

H ; - ; - ; Phe ; H ; H ; $NH_2$-$CH_2$-C(O)-NH-$CH_2$-C(O)-NH-$CH_2$-C(O)- ; O ; 2 ; Me ; Me ;

H ; - ; - ; néopentyl ; H ; H ; aminohexylcarbonyl ; O ; 1 ; H ; Phe ;

H ; - ; - ; isobutyl ; H ; H ; aminohexylcarbonyl ; O ; 1 ; H ; Phe ;

H ; - ; - ; isobutyl ; H ; H ; H ; O ; 1 ; H ; Phe ;

$R'_1$-NH-C(Y)- ; 2-$NO_2$-4-MeO-Phe ; S ; 2-Cl-Phe ; H ; H ; H ; O ; 4 ; H ; H ;

$R'_1$-NH-C(Y)- ; 2-$NO_2$-4-MeO-Phe ; S ; 2-Cl-Phe ; H ; H ; -$CH_2CH=C(Me)_2$ ; O ; 1 ; H ; Phe ;

$R'_1$-NH-C(Y)- ; 2-$NO_2$-4-MeO-Phe ; S ; 2-Cl-Phe ; H ; H ; aminohexylcarbonyl ; O ; 1 ; H ; Phe ;

$R'_1$-NH-C(Y)- ; 2-$NO_2$-4-MeO-Phe ; S ; 2-Cl-Phe ; H ; H ; propylcarbonyl ; O ; 1 ; H ; Phe ;

$R'_1$-NH-C(Y)- ; 2-$NO_2$-4-MeO-Phe ; S ; 2-Cl-Phe ; H ; H ; cyclopentyl-méthylcarbonyl ; O ; 1 ; H ; Phe ;

$R'_1$-NH-C(Y)- ; 2-$NO_2$-4-MeO-Phe ; S ; 2-Cl-Phe ; H ; H ; phényl-propylcarbonyl ; O ; 1 ; H ; Phe ;

$R'_1$-NH-C(Y)- ; 2-$NO_2$-4-MeO-Phe ; S ; 2-Cl-Phe ; H ; H ; phényléthylcarbonyl ; O ; 1 ; H ; Phe ;

$R'_1$-NH-C(Y)- ; 2-$NO_2$-4-MeO-Phe ; S ; 2-Cl-Phe ; H ; H ; aminobutylcarbonyl ; O ; 1 ; H ; Phe ;

$R'_1$-NH-C(Y)- ; 2-$NO_2$-4-MeO-Phe ; S ; 2-Cl-Phe ; H ; H ; indolylméthylcarbonyl ; O ; 1 ; H ; Phe ;

$R'_1$-NH-C(Y)- ; 2-$NO_2$-4-MeO-Phe ; S ; 2-Cl-Phe ; H ; H ; aminopentylcarbonyl ; O ; 1 ; H ; Phe ;

$R'_1$-NH-C(Y)- ; 2-$NO_2$-4-MeO-Phe ; S ; Phe ; H ; H ; $NH_2$-$CH_2$-C(O)-NH-$CH_2$-C(O)-NH-$CH_2$-C(O)- ; O ; 2 ; Me ; Me ;

$R'_1$-NH-C(Y)- ; 2-$NO_2$-4-MeO-Phe ; S ; Phe ; H ; H ; aminohexylcarbonyl ; O ; 2 ; Me ; Me ;

$R'_1$-NH-C(Y)- ; 2-$NO_2$-4-MeO-Phe ; S ; néopentyl ; H ; H ; aminohexylcarbonyl; O ; 1 ; H ; Phe ;

$R'_1$-NH-C(Y)- ; 2-$NO_2$-4-MeO-Phe ; S ; isobutyl ; H ; H ; aminohexylcarbonyl ; O ; 1 ; H ; Phe.

Un composé de formule (I) selon l'invention et dans laquelle A---B représente C=N, $R_1$ l'atome d'hydrogène et $R_{3a}$ l'atome d'hydrogène ou un radical alkyle, peut être obtenu en faisant réagir un composé de formule (1)

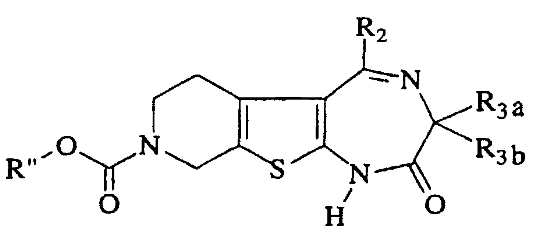

(1)

dans laquelle $R_2$, $R_{3a}$, $R_{3b}$ ont la signification indiquée ci-dessus et R" représente un radical alkyle inférieur ou arylalkyle inférieur, avec un composé $R_4Z$ dans lequel $R_4$ a la signification indiquée ci-dessus et Z représente un groupe partant, en présence d'une base forte, pour obtenir le composé (2)

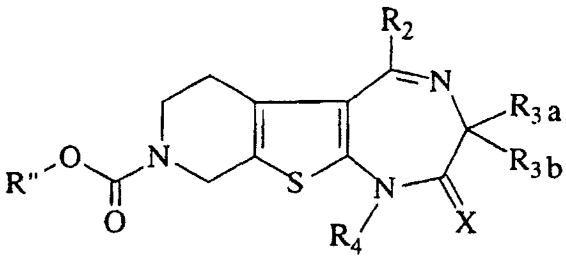

(2)

dans lequel X représente l'atome d'oxygène, composé (2) ainsi obtenu que l'on peut faire réagir avec un réactif de thiation pour obtenir le composé (2) dans lequel X représente un atome de soufre,

composé (2) dans lequel X représente un atome d'oxygène ou de soufre, qui est enfin soumis à une réaction de déprotection du carbamate pour obtenir le produit (I) souhaité.

Lors de la préparation des composés de formule (2) dans laquelle X représente l'atome d'oxygène, les composés de formule (1) sont soumis à l'action d'une base forte comme par exemple l'hydrure de sodium au sein d'un solvant inerte comme par exemple le tétrahydrofurane ou la diméthylformamide à une température proche de 20° C. Le composé $R_4Z$ est ensuite ajouté au milieu réactionnel à une température proche de 20° C puis le milieu réactionnel est chauffé aux environs de 80° C. Le groupe partant Z du composé $R_4Z$, peut être par exemple un mésylate, tosylate ou un atome d'halogène (de préférence un atome de chlore ou de brome). La préparation des composés de formule (2) dans laquelle X représente l'atome de soufre à partir des composés de formule (2) dans laquelle X représente l'atome d'oxygène, peut être mise en oeuvre à une température proche de 80° C avec un agent de thiation comme le pentasulfure de phosphore dans un solvant comme la pyridine.

La déprotection du carbamate qui ne touche pas le reste de la molécule, peut-être effectuée selon les méthodes connues de déprotection (T.W. Greene et al., Protective Groups in Organic Synthesis, Wiley-Interscience, 1991). Ainsi, dans le cas où R" représente un groupement alkyle linéaire (tel que éthyle) ou arylalkyle (tel que benzyle), la déprotection du carbamate peut être mise en oeuvre en agitant le milieu réactionnel à température ambiante en milieu fortement acide comme, par exemple, dans l'acide bromhydrique (33 % dans de l'acide acétique). Dans le cas où R" représente un groupement alkyle plus encombré (tel que t-butyle), la réaction peut être mise en oeuvre dans l'acide trifluoroacétique dans un solvant inerte tel que le dichlorométhane à une température voisine de 20° C.

Les produits de formule (1) peuvent-être préparés selon la méthode décrite dans le brevet FR 2645153 ou selon des méthodes analogues.

Certains des produits de formule $R_4Z$ sont en général commercialisés (par exemple par les firmes Acros ou Aldrich) ; les autres peuvent être préparés à partir de l'alcool de formule $R_4$-OH dans un solvant inerte comme le dichlorométhane par action par exemple du chlorure de tosyle en présence de triéthylamine ou bien par action de la triphénylphosphine et du tétrabromure de carbone.

Un composé de formule (I) selon l'invention et dans laquelle A---B représente C=N, $R_1$ l'atome d'hydrogène et $R_{3a}$ le radical hydroxy, peut être obtenu par oxydation d'un composé de formule (2) tel que défini ci-dessus, dans un solvant inerte pour obtenir un composé de formule (3)

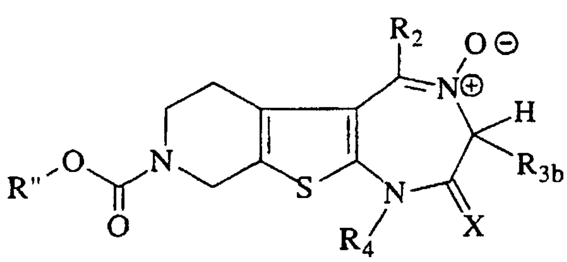

(3)

dans laquelle $R_2$, $R_{3b}$, $R_4$, R" et X ont la signification indiquée ci-dessus,

composé de formule (3) que l'on traite avec l'anhydride acétique pour obtenir un composé de formule (4)

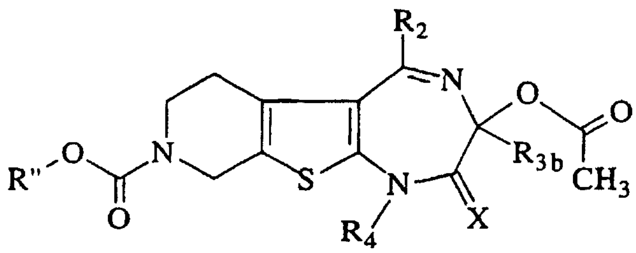

(4)

dans laquelle $R_2$, $R_{3b}$, $R_4$, R" et X ont la signification indiquée ci-dessus,
composé (4) qui est ensuite saponifié pour obtenir le composé de formule (5)

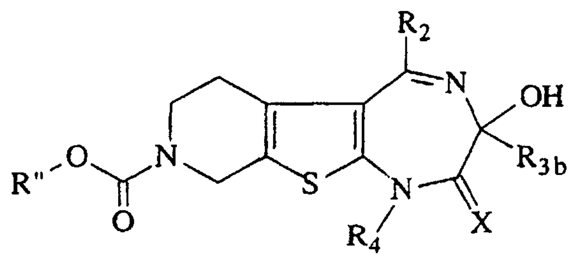

(5)

dans laquelle $R_2$, $R_{3b}$, $R_4$, R" et X ont la signification indiquée ci-dessus,
composé (5) qui est enfin soumis à une réaction de déprotection du carbamate pour obtenir le composé correspondant de formule (I) dans laquelle $R_1$ représente H et $R_{3a}$ le radical hydroxy.

L'oxydation du composé (2) au niveau de l'imine de la diazépine, peut s'effectuer par action d'un oxydant organique comme par exemple l'acide métachloroperoxybenzoïque, à une température voisine de 20° C, dans un solvant inerte comme le dichlorométhane ou le 1,2-dichloroéthane. La réaction du composé (3) avec l'anhydride acétique est un réarrangement de type Polonowski (Gilman N. W. et al., J. Am. Chem. Soc. 1990, 112, 3969-3978) qui peut être mise en oeuvre à une température voisine de 70° C. La réaction de saponification du composé (4) peut s'effectuer par action d'une base minérale comme par exemple l'hydroxyde de sodium ou l'hydroxyde de lithium, dans un alcool aliphatique inférieur (méthanol, éthanol par exemple), à une température voisine de 20° C.

Un composé de formule (I) selon l'invention et dans laquelle A---B représente C=N, $R_1$ l'atome d'hydrogène et $R_{3a}$ un radical -OC(O)-$R'_{3a}$, peut être obtenu en faisant réagir un composé de formule (5) tel que défini ci-dessus, avec un acide de formule $R'_{3a}$C(O)OH dans laquelle $R'_{3a}$ a la signification indiquée ci-dessus, pour obtenir un composé de formule (6)

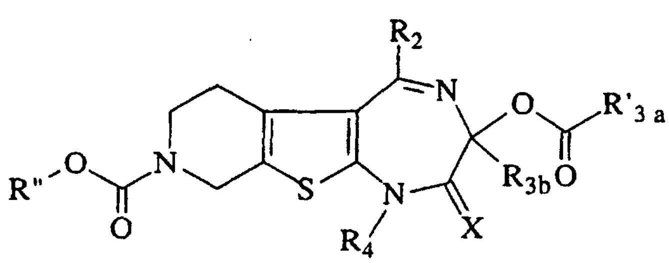

(6)

dans laquelle $R_2$, $R'_{3a}$, $R_{3b}$, $R_4$, R" et X ont la signification indiquée ci-dessus,
composé (6) qui est enfin soumis à une réaction de déprotection du carbamate pour obtenir le composé correspondant de formule (I) dans laquelle $R_1$ représente H et $R_{3a}$ le radical -OC(O)-$R'_{3a}$.

La transformation du composé (5) en composé (6) peut s'effectuer dans des conditions analogues à celles de réactions d'estérification, connues de l'homme de l'art ; elle peut ainsi être mise en oeuvre à une température voisine

de 20° C dans un solvant inerte comme le dichlorométhane ou le 1,2-dichloroéthane.

Les composés de formule I selon l'invention dans laquelle A---B représente C=N, $R_1$ un radical de formule $R'_1$-NH-C(Y)-, peuvent être préparés selon le procédé qui consiste à faire réagir le composé correspondant de formule (I) dans laquelle $R_1$ représente l'atome d'hydrogène, avec un composé de formule

$$R'_1\text{-N=C=Y} \quad (7)$$

dans laquelle $R'_1$ et Y ont la signification indiquée ci-dessus, pour former le composé de formule I choisi.

Lors de la préparation du composé de formule I dans laquelle $R_1$ représente un radical de formule $R'_1$-NH-C(Y)-, l'addition du composé de formule (7), sur le composé de formule (I) dans laquelle $R_1$ représente l'atome d'hydrogène, se fait aisément à une température voisine de 20° C dans un solvant chloré comme le dichlorométhane ou le 1,2-dichloroéthane. Les produits de formule (7) sont pour la plupart commerciaux ou peuvent-être préparés en faisant réagir l'amine correspondante sur le (thio)phosgène selon les méthodes connues de l'homme de l'art.

Un composé de formule (I) selon l'invention et dans laquelle A---B représente -C-N($R_5$)-, $R_1$ et $R_5$ un atome d'hydrogène et $R_{3a}$ l'atome d'hydrogène ou un radical alkyle, peut être obtenu par action d'un agent réducteur doux en milieu acide, sur un composé de formule (2) tel que défini ci-dessus, pour obtenir le composé de formule (8)

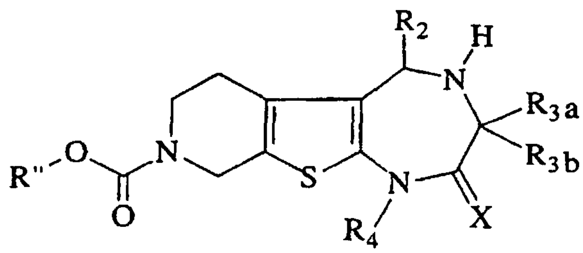

(8)

dans laquelle $R_2$, $R_{3a}$, $R_{3b}$, $R_4$, X et R" ont la signification indiquée ci-dessus, composé (8) qui est soumis à une réaction de déprotection du carbamate pour obtenir le produit (I) souhaité dans lequel $R_1$ représente l'atome d'hydrogène.

Lors de la réduction du composé (2) pour obtenir le composé (8), on peut utiliser un agent réducteur doux comme le cyanoborohydrure de sodium dans un solvant tel qu'un solvant alcoolique inférieur (méthanol, éthanol par exemple) à une température voisine de 20° C.

Un composé de formule (I) selon l'invention et dans laquelle A---B représente C-$NR_5$, $R_1$ représente un atome d'hydrogène, $R_{3a}$ l'atome d'hydrogène ou le radical alkyle et $R_5$ représente un radical alkenyle, peut être obtenu en faisant réagir un composé de formule (8) tel que défini ci-dessus, avec un composé de formule $Z'R_5$ dans laquelle $R_5$ a la signification indiquée ci-dessus et Z' représente un groupe partant, en présence d'une base minérale forte dans un solvant inerte, pour obtenir le composé (9)

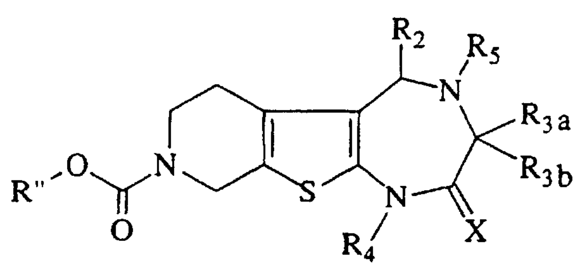

(9)

dans lequel $R_2$, $R_{3a}$, $R_{3b}$, $R_4$, X, R" et $R_5$ ont la signification indiquée ci-dessus, composé (9) qui est soumis à une réaction de déprotection du carbamate pour obtenir le produit (I) souhaité dans lequel $R_1$ représente l'atome d'hydrogène.

La préparation du composé (9) à partir du composé (8), peut être mise en oeuvre par action d'hydrure de sodium dans un solvant tel que le tétrahydrofurane à une température voisine de 60° C. Le groupe partant Z' du composé $Z'R_5$ peut être un mésylate, tosylate ou un atome d'halogène.

Un composé de formule (I) selon l'invention et dans laquelle A---B représente -C-N($R_5$)-, $R_1$ représente un atome d'hydrogène, $R_{3a}$ l'atome d'hydrogène ou le radical alkyle et $R_5$ représente un radical -C(O)-$(CH_2)_p$-$R'_5$, peut être obtenu en faisant réagir un composé de formule (8) tel que défini ci-dessus, avec un acide de formule $R'_5$-$(CH_2)_p$-C(O)OH dans laquelle $R'_5$ et p ont la signification indiquée ci-dessus, pour obtenir le composé (10) correspondant,

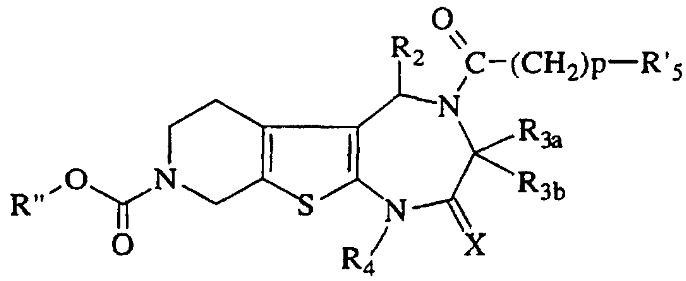

(10)

dans lequel $R_2$, $R_{3a}$, $R_{3b}$, $R_4$, X, R", p et $R'_5$ ont la signification indiquée ci-dessus,
composé (10) qui est soumis à une réaction de déprotection du carbamate pour obtenir le produit (I) souhaité dans lequel $R_1$ représente l'atome d'hydrogène.

La réaction du composé $R'_5$-$(CH_2)_p$-C(O)OH sur le composé (8) peut s'effectuer dans des conditions similaires aux réactions de couplage peptidique. Elle peut être mise en oeuvre à une température voisine de 20° C dans un solvant inerte tel que le dichlorométhane ou le 1,2-dichloroéthane.

Un composé de formule (I) selon l'invention et dans laquelle A---B représente -C-N($R_5$)-, $R_1$ l'atome d'hydrogène et $R_{3a}$ le radical hydroxy, peut être obtenu par réduction du composé de formule (4), pour obtenir le composé de formule (11)

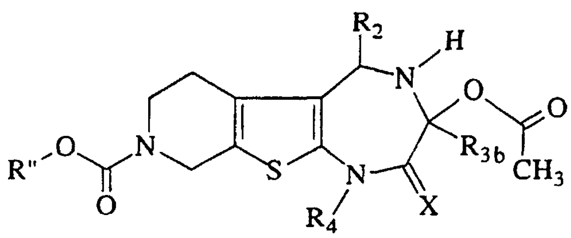

(11)

dans laquelle $R_2$, $R_{3b}$, $R_4$, X et R" ont la signification indiquée ci-dessus,
composé (11) qui est

- soit soumis à une réaction de saponification puis de déprotection du carbamate pour obtenir le produit (I) souhaité dans lequel $R_1$ et $R_5$ représentent l'atome d'hydrogène ;

- soit traité par un composé de formule halo-$R_5$ dans laquelle $R_5$ représente un radical alkenyle ou un acide de formule $R'_5$-$(CH_2)_p$-C(O)OH dans laquelle $R'_5$ a la signification indiquée ci-dessus, pour obtenir le composé de formule (12)

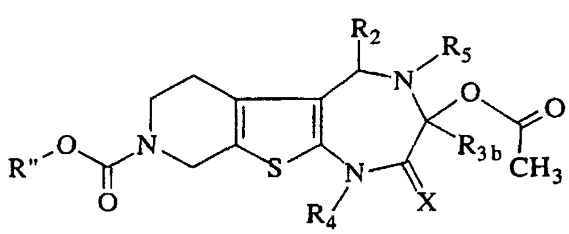

(12)

dans laquelle $R_2$, $R_{3b}$, $R_4$, X et R" ont la signification indiquée ci-dessus et $R_5$ représente respectivement un radical

alkenyle ou $R'_5$-$(CH_2)_p$-C(O)-,
composé (12) qui est enfin soumis à une réaction de saponification puis de déprotection du carbamate pour obtenir le produit (I) souhaité dans lequel $R_1$ représente l'atome d'hydrogène et $R_5$ un radical alkenyle ou $R'_5$-$(CH_2)_p$-C(O)-.

Un composé de formule (I) selon l'invention et dans laquelle A---B représente -C-N($R_5$)-, $R_1$ l'atome d'hydrogène et $R_{3a}$ un radical -OC(O)-$R'_{3a}$, peut être obtenu par réduction du composé de formule (6), pour obtenir un composé de formule (13)

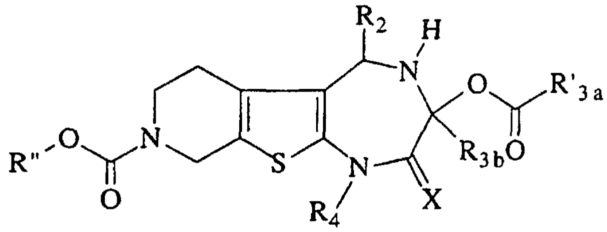

(13)

dans laquelle $R_2$, $R'_{3a}$, $R_{3b}$, $R_4$, X et R" ont la signification indiquée ci-dessus,
composé (13) qui est

- soit soumis à une réaction de déprotection du carbamate pour obtenir le produit (I) souhaité dans lequel $R_1$ et $R_5$ représentent l'atome d'hydrogène ;
- soit traité par un composé halo-$R_5$ dans laquelle $R_5$ représente un radical alkenyle ou un acide de formule $R'_5$-$(CH_2)_p$-C(O)OH dans laquelle $R'_5$ a la signification indiquée ci-dessus, pour obtenir le composé de formule (14)

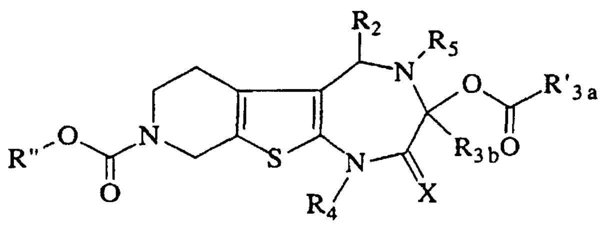

(14)

dans laquelle $R_2$, $R'_{3a}$, $R_{3b}$ , $R_4$, X et R" ont la signification indiquée ci-dessus et $R_5$ représente respectivement un radical alkenyle ou $R'_5$-$(CH_2)_p$-C(O)- ;
composé (14) qui est enfin soumis à une réaction de déprotection du carbamate pour obtenir le produit (I) souhaité dans lequel $R_1$ représentent l'atome d'hydrogène et $R_5$ respectivement un radical alkenyle ou $R'_5$-$(CH_2)_p$-C(O)-.

Les conditions d'ajout du radical $R_5$ (différent de H) sur l'atome d'azote des diazépines (11) et (13) sont identiques aux conditions de réactions pour la préparation des composés (9) et (10). Les réactions de déprotection des carbamates (5), (6), (8), (9), (10), (13) et (14) sont telles que définies précédemment ; le radical R" est choisi suivant les autres fonctions présentes dans la molécule et dans le but d'obtenir une déprotection sélective du groupement carbamate R"OC(O)N-.

Les composés de formule I selon l'invention dans laquelle A---B représente -C-N($R_5$)-et $R_1$ représente un radical de formule $R'_1$-NH-C(Y)-, peuvent être préparés par action d'un composé de formule (7) $R'_1$-N=C=Y dans laquelle $R'_1$ et Y ont la signification indiquée ci-dessus, sur le composé de formule (I) correspondant dans laquelle $R_1$ représente l'atome d'hydrogène, pour former le composé de formule I choisi.

Les composés de formule (2) sont nouveaux. L'invention a également pour objet, à titre de produits industriels nouveaux, et notamment à titre de produits industriels nouveaux destinés à la préparation des composés de formule (I) selon l'invention, les produits de formule (2).

Les composés I de la présente invention possèdent d'intéressantes propriétés pharmacologiques. C'est ainsi que l'on a découvert que les composés I de la présente invention ont une haute affinité pour un (ou plusieurs) des récepteurs de la somatostatine. Ils peuvent être utilisés comme agonistes ou antagonistes non-peptidiques de la somatostatine de manière sélective ou non.

Les composés de la présente invention peuvent ainsi être utilisés dans différentes applications thérapeutiques. Ils peuvent avantageusement être utilisés pour traiter les états pathologiques ou les maladies tels que présentés

ci-dessus et dans lesquels un (ou plusieurs) des récepteurs de la somatostatine est (sont) impliqué(s).

On trouvera ci-après, dans la partie expérimentale, une illustration des propriétés pharmacologiques des composés de l'invention.

La présente demande a également pour objet, à titre de médicaments, les produits de formule I telle que définie ci-dessus, ainsi que les sels d'addition avec les acides minéraux ou organiques pharmaceutiquement acceptables desdits produits de formule I, ainsi que les compositions pharmaceutiques contenant, à titre de principe actif, un au moins des médicaments tels que définis ci-dessus, en association avec un support pharmaceutiquement acceptable.

La composition pharmaceutique peut être sous forme d'un solide, par exemple des poudres, des granules, des comprimés, des gélules ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau, additionnés à des huiles ou des graisses pharmaceutiquement acceptables. Les compositions liquides stériles peuvent être utilisées pour les injections intramusculaires, intrapéritonéales ou sous-cutanées et les compositions stériles peuvent également être administrées par intraveineuse.

Tous les termes techniques et scientifiques utilisés dans le présent texte ont la signification connue de l'homme de l'art. Par ailleurs, tous les brevets (ou demandes de brevet) ainsi que les autres références bibliographiques sont incorporés par référence.

Les exemples suivants sont présentés pour illustrer les procédures ci-dessus et ne doivent en aucun cas être considérés comme une limite à la portée de l'invention.

## PARTIE EXPÉRIMENTALE :

### Exemple 1

5-(2-chlorophényl)-1,3,6,7,8,9-hexahydro-1-(2-phényléthyl)-2H-pyrido [4',3':4,5] thiéno [2,3-e]-1,4-diazépine-2-one

1ère étape

5-(2-chlorophényl)-1,2,3,6,7,9-hexahydro-2-oxo-1-(2-phényléthyl)-8H-pyrido[4',3':4,5] thiéno [2,3-e] -1,4-diazépine-8-carboxylate d'éthyle

Sous argon, on solubilise 18 g de 5-(2-chlorophényl)-1,2,3,6,7,9-hexahydro-2-oxo-8H-pyrido [4',3':4,5]thiéno [2,3-e]-1,4-diazépine-8-carboxylate d'éthyle (4,46 mmol) dans 180 ml de diméthylformamide anhydre. On ajoute de l'hydrure de sodium à 60 % (1,784 g, 4,46 mmol) puis on chauffe environ 30 minutes à 50° C jusqu'à ce qu'il n'y ait plus de dégagement. On refroidit à une température voisine de 20° C puis on ajoute à la seringue le 2-bromoéthylbenzène (6,2 ml, 4,46 mmol). On agite 16 heures à 23° C puis on verse le milieu réactionnel dans une solution saturée en chlorure d'ammonium (400ml). On extrait avec de l'acétate d'éthyle (2 x 500 ml). La phase organique est séchée sur sulfate de magnésium puis le solvant est évaporé au rotavapor. Après purification par chromatographie sur colonne de silice (éluant : acétate d'éthyle-heptane : 0-100 à 20-80), on évapore des fractions contenant seulement le produit et on obtient le produit souhaité sous forme d'une poudre amorphe blanche (20,3 g, 89 %).

Point de fusion : 70-78° C

RMN$^1$H (400 MHz, $CDCl_3$, δ) : 1,07 (m, 3H) ; 1,46 (m, 1H) ; 1,93-1,97 (m, 1H) ; 2,85 (m, 2H) ; 3,08 (m, 1H) ; 3,69 (m, 2H) ; 3,69 (m, 1H) ; 4,02 (m, 2H) ; 4,38 (m, 2H) ; 4,73 (m, 2H) ; 7,20-7,49 (m, 9H).

IR ($cm^{-1}$) : 3427 ; 2978 : 2927 ; 1686 (V C=O carbamate) ; 1678 (V lactame) ; 1231 ; 1115 ; 761

2ème étape

5-(2-chlorophényl)-1,3,6,7,8,9-hexahydro-1-(2-phényléthyl)-2H-pyrido [4',3':4,5]thiéno [2,3-e]-1,4-diazépine-2-one

On agite durant 12 heures à une température voisine de 20° C, un mélange contenant le composé obtenu à l'étape précédente (20,3 g, 0,04 mol) dans une solution d'acide bromhydrique à 33 % dans l'acide acétique (500 ml). On chauffe 3 heures à 40° C puis on évapore les acides au rotavapor. L'huile obtenue est reprise dans 300 ml d'eau puis une solution saturée en bicarbonate de sodium (300 ml) est ajoutée doucement et avec précaution. On extrait avec du dichlorométhane (2 x 300 ml), sèche sur sulfate de magnésium et évapore le solvant pour obtenir le produit

souhaité sous forme d'une poudre amorphe violacée (17 g, 97 %).
Point de fusion : 70-78° C
IR ($cm^{-1}$) : 3427 ; 2978 ; 2927 ; 1678 (V lactame) ; 1231 ; 1115 ; 761
HPLC (UV) : 99 %

**Exemple 2**

5-(2-chlorophényl)-1,2,3,6,7,9-hexahydro-N-(4-méthoxy-2-nitrophényl)-2-oxo-1-(2-phényl-éthyl)-8H-pyrido [4',3':4,5]thiéno[2,3-e]-1,4-diazépine-8-carbothioamide

On agite durant 3 heures à une température voisine de 20° C et sous atmosphère d'argon, un mélange contenant la 5-(2-chlorophényl)-1,3,6,7,8,9-hexahydro-1-(2-phényléthyl)-2H-pyrido[4',3':4,5]thiéno[2,3-e]-1,4-diazépine-2-one (0,2 g, 0,46 mmol) et le 4-méthoxy-2-nitrophenylisothiocyanate (0,096 g, 0,46 mmol) dans 4 ml de dichlorométhane anhydre. On ajoute dans le milieu réactionnel de l'éther (2 ml) et on laisse l'agitation environ trente minutes jusqu'à précipitation. Le solide est filtré sur fritté et lave à l'éther isopropylique (2 x 5 ml) puis à l'isopentane (5 ml). Après séchage sous vide à une température inférieure à 100° C, on obtient le produit souhaité sous forme d'une poudre jaune (0,195 g, 66 %).
Point de fusion : 145-150° C
RMN$^1$H (400 MHz, $CDCl_3$, δ) : 1,70 (m, 1H) ; 2,12 (m, 1H) ; 2,81-2,88 (m, 2H) ; 3,55 (m, 1H) ; 3,74 (m, 1H) ; 3,84 (s, 3H) ; 3,96 (m, 1H) ; 4,23 (m, 1H) ; 4,41 (m, 1H) ; 4,73-4,82 (m, 2H) ; 5,42 (m, 1H) ; 7,20-7,51 (m, 12H) ; 9,49 (s, 1H).
IR ($cm^{-1}$) : 3427 ; 2978 ; 2927 ; 1678 (V C=O lactame) ; 1530 ; 1210 ; 1030
HPLC (UV) : 99 %

**Exemple 3**

5-(2-chlorophényl)-1,3,6,7,8,9-hexahydro-1-(2-phényléthyl)-2H-pyrido [4',3:4,5]thiéno [2,3-e] -1,4-diazépine-2-thione

1ère étape

5-(2-chlorophényl)-1,2,3,6,7,9-hexahydro-1-(2-phényléthyl)-2-thioxo-8H-pyrido[4',3':4,5] thiéno[2,3-e]-1,4-diazépine-8-carboxylate d'éthyle

On chauffe à une température voisine de 85° C et durant 5 heures un mélange contenant le 5-(2-chlorophényl)-1,2,3,6,7,9-hexahydro-2-oxo-1-(2-phényléthyl)-8H-pyrido [4',3':4,5] thieno [2,3-e]-1,4-diazépine-8-carboxylate d'éthyle (0,97 g, 1,91 mmol) et du pentasulfure de phosphore (0,425 g, 1,91 mmol) dans de la pyridine (10 ml). On revient à une température voisine de 20° C et le résidu est filtré sur fritté. La pyridine contenu dans le filtrat est évaporée au rotavapor (en s'aidant avec du toluène). On ajoute ensuite du dichlorométhane (40 ml) puis la phase organique est lavée, séchée sur sulfate de magnésium et le solvant évaporé. On chauffe ensuite la poudre obtenue pendant une heure au reflux de l'acétonitrile (100 ml). Après évaporation du solvant au rotavapor, on purifie par chromatographie sur colonne de silice (éluant : acétate d'éthyle-heptane : 20-80 à 45-55). On évapore les fractions contenant seulement le produit pour obtenir le produit souhaité sous forme d'une poudre jaune (0,1 g).
RMN$^1$H (400 MHz, $CDCl_3$, δ) : 0,84 (m, 3H) ; 1,49 (m, 1H) ; 1,95-1,99 (m, 1H) ; 2,8-3,12 (m, 3H) ; 3,70 (m, 2H) ; 4,03-4,19 (m, 2H) ; 4,31-4,34 (m, 2H) ; 4,80 (m, 1H) ; 4,98-5,05 (m, 1H) ; 5,37 (d, 1H) ; 6,95-7,67 (m,9H)
IR ($cm^{-1}$) : 3427 ; 2978 ; 2927 ; 1701 (V C=O lactame) ; 1592 ; 1384 ; 1296 ; 1225 ; 761

2ème étape

5-(2-chlorophényl)-1,3,6,7,8,9-hexahydro-1-(2-phényléthyl)-2H-pyrido [4',3':4,5] thiéno [2,3-e]-1,4-diazépine-2-thione

On agite, pendant trois jours, le composé obtenu lors de l'étape précédente (0,1 g, 0,19 mmol) dans de l'acide bromhydrique dilué à 33 % dans l'acide acétique (30 ml). On évapore au rotavapor les acides en s'aidant avec du toluène. On ajoute du dichlorométhane (30 ml) et une solution saturée en bicarbonate de sodium (50 ml). On agite durant quelques heures puis la phase organique est extraite et séchée sur sulfate de magnésium et le solvant est évaporé pour obtenir le produit souhaité.

**Exemple 4**

5-(2-chlorophényl)-1,2,3,6,7,9-hexahydro-N-(4-méthoxy-2-nitrophényl)-1-(2-phényléthyl)-2-thioxo-8H-pyrido[4',3:4,5] thiéno[2,3-e]-1,4-diazépine-8-carbothioamide

86 mg de 5-(2-chlorophényl)-1,3,6,7,8,9-hexahydro-1-(2-phényléthyl)-2H-pyrido [4',3':4,5] thiéno [2,3-e]-1,4-diazépine-2-thione sont mis en réaction avec du 4-méthoxy-2-nitroisothiocyanate (0,040 g, 0,19 mmol) dans 2 ml de dichlorométhane à une température voisine de 20° C. Le mélange réactionnel est agité durant 2 heures et de l'éther isopropylique (3 ml) est ajouté jusqu'à précipitation. Après filtration sur fritté et lavage à l'éther isopropylique (2 x 3 ml) et à l'isopentane (5 ml), on purifie par chromatographie sur colonne de silice (éluant : acétate d'éthyle-heptane : 20-80 à 40-60). Après évaporation des fractions contenant seulement le produit, on obtient le produit souhaité sous forme d'une poudre orange (0,03 g, 24 %).
Point de fusion : 152°C
HPLC MS : 99 % à 230 nm (MH+ trouvée 662,1 ; MH+ théorie 662,2)

**Exemple 5**

5-(2-chlorophényl)-1,2,3,6,7,9-hexahydro-3-hydroxy-1-(2-phényléthyl)-8H-pyrido [4',3':4,5] thieno[2,3-e]-1,4-diazépine-2-one

1ère étape

5-(2-chlorophenyl)-1,2,3,6,7,9-hexahydro-4-oxide-2-oxo-1-(2-phényléthyl)-8H-pyrido [4',3':4,5] thieno [2,3-e]-1,4-diazépine-8-carboxylate de t-butyle

On dissout à 23° C du 5-(2-chlorophényl)-1,2,3,6,7,9-hexahydro-2-oxo-1-(2-phényléthyl)-8H-pyrido[4',3':4,5] thiéno[2,3-e]-1,4-diazépine-8-carboxylate de t-butyle (22 g, 41 mmol) dans du 1,2-dichloro éthane (320 ml). On ajoute de l'acide méta-chloroperoxybenzoique à 85 % (21,6 g, 0,1 mol). On agite 24 h à 23° C puis on ajoute une solution d'hydroxyde de sodium 5N (400 ml). Après décantation, on lave la phase organique à l'eau, sèche sur sulfate de magnésium, filtre et concentre au rotavapor. Après purification par chromatographie sur colonne de silice (éluant : acétate d'éthyle-heptane : 50-50 à 80-20), on évapore les fractions contenant seulement le produit. Le solide obtenu est agité dans un mélange de solvants : éther isopropylique-isopentane (20-80). Après filtration sur fritté et lavage à l'isopentane, on obtient le produit souhaité sous forme d'une poudre blanche (5,9 g, 26 %).
RMN$^1$H (400 MHz, $CDCl_3$, δ) : 1,35 (s, 9H) ; 1,96 (m, 1H); 2,87-3,07 (m, 3H) ; 3,70 (m, 1H) ; 3,88 (m, 1H) ; 4,30 (m, 1H) ; 4,45 (m, 2H) ; 4,67 (m, 1H) ; 4,99 (m, 1H) ; 6,81 (s, 1H) ; 7,22-7,84 (m, 9H).
IR ($cm^{-1}$) : 1689 (ν C=O carbamate) ; 1679 (ν C=O lactame) ; 750
HPLC (UV) : 99 %

2eme étape

3-acétyloxy-5-(2-chlorophényl)-1,2,3,6,7,9-hexahydro-2-oxo-1-(2-phényléthyl)-8H-pyrido [4'.3':4,5]thiéno [2,3-e]-1,4-diazépine-8-carboxylate de t-butyle

On verse le composé obtenu à l'étape précédente (5,8 g, 10 mmol) dans de l'anhydride acétique (58 ml) puis on chauffe à 70° C pendant 2 h, On laisse refroidir puis on verse le milieu réactionnel dans une solution saturée en bicarbonate de sodium à 10 % (150 ml). On agite 30 minutes puis on extrait à l'acétate d'éthyle. On décante, lave la phase organique avec une solution saturée en bicarbonate de sodium à 10 % (2 x 150 ml) puis à l'eau (2 x 150 ml). La phase organique est séchée sur sulfate de magnésium et le solvant est évaporé. Après purification par chromatographie sur colonne de silice (éluant : acétate d'éthyle-heptane : 20-80 à 50-50), on évapore les fractions contenant seulement le produit. Le solide obtenu est retraité dans un mélange de solvants : éther isopropylique-isopentane (20-80). Après filtration sur fritté et lavage à l'isopentane, on obtient le produit souhaité sous forme d'une poudre beige (5,2 g, 84 %).
**IR** ($cm^{-1}$) : 3420 ; 2975 ; 1742 (ν C=O ester) ; 1702 (ν C=O carbamate) ; 1679 (ν lactame) ; 1233 ; 1111 ; 755

3ème étape

5-(2-chlorophényl)-1,2,3,6,7,9-hexahydro-3-hydroxy-2-oxo-1-(2-phényléthyl)-8H-pyrido[4',3':4,5]thiéno [2,3-e]-1,4-diazépine-8-carboxylate de t-butyle

On dissout le composé obtenu à l'étape précédente (2,5 g, 4,2 mmol) dans du méthanol (60 ml). On refroidit à -5° C puis on ajoute goutte à goutte une solution d'hydroxyde de sodium (0,168 g dans 6 ml d'eau). On agite 2 h à 23° C. Le solvant est évaporé puis le mélange réactionnel est repris dans du dichlorométhane. On lave à l'eau puis avec une solution saturée en chlorure d'ammonium (2 x 100 ml). Après décantation, la phase organique est lavée à l'eau, séchée sur sulfate de magnésium et le solvant est évaporé. Après purification par chromatographie sur colonne de silice (éluant : acétate d'éthyle-heptane : 20-80 à 50-50), on évapore les fractions contenant seulement le produit. Après filtration sur fritté et lavage à l'isopentane, on obtient le produit souhaité sous forme d'une poudre jaune (4,1 g,

87 %).
Point de fusion : 138-140° C

4ème étape

5-(2-chlorophényl)-1,2,3,6,7,9-hexahydro-3-hydroxy-1-(2-phényléthyl)-8H-pyrido [4',3':4,5]thieno[2,3-e]-1,4-diazépine-2-one

Le produit obtenu à l'étape précédente (0,56 g, 1 mmol) est solubilisé dans du dichlorométhane (8 ml). On ajoute l'acide trifluoroacétique (2,5 ml) et on agite 2 h à 23° C. Le solvant est évaporé et l'huile obtenue est reprise dans 5 ml d'acétone. On ajoute 20 ml d'éther et on agite jusqu'à précipitation. Après filtration sur fritté et lavage à l'éther, on obtient le produit souhaité sous forme d'une poudre beige (0,32 g, 56 %).
Point de fusion : 180-189° C

**Exemple 6**

5-(2-chlorophenyl)-1,2,3,6,7,9-hexahydro-3-hydroxy-N-(4-méthoxy-2-nitrophényl)-2-oxo-1-(2-phényléthyl)-8H-pyrido[4',3':4,5]thiéno[2,3-e]-1,4-diazépine-8-carbothioamide

On dissout la 5-(2-chlorophényl)-1,2,3,6,7,9-hexahydro-3-hydroxy-1-(2-phényléthyl)-8H-pyrido[4',3':4,5]thiéno[2,3-e]-1,4-diazépine-2-one (0,167 g, 0,29 mmol) dans du dichlorométhane (2 ml). On ajoute de la triéthylamine (0,06 ml, 0,44 mmol) puis le 4-méthoxy-2-nitrophénylisothiocyanate (0,062 g, 0,29 mmol). Après agitation à 23° C durant 2 h, on évapore le solvant. Après purification par chromatographie sur colonne de silice (éluant : dichlorométhane-méthanol: 98-2), on évapore les fractions contenant seulement le produit. Le solide est repris dans de l'éther et une goutte de dichlorométhane et une goutte d'acétone sont rajoutées. Après filtration sur fritté et lavage du solide à l'éther, on obtient le produit souhaité sous forme d'une poudre jaune (0,08 g, 41 %).
Point de fusion : 143-145° C

**Exemple 7**

3-(7-amino-1-oxo-heptyloxy)-5-(2-chlorophényl)-1,2,3,6,7,9-hexahydro-2-oxo-1-(2-phényléthyl)-8H-pyrido [4',3':4,5]thiéno[2,3-e]-1,4-diazépine

1ère étape

5-(2-chlorophényl)-3-[7-[(1,1-diméthyléthoxycarbonyl)amino]-1-oxo-heptyloxy]-1,2,3,6,7,9-hexahydro-2-oxo-1-(2-phényléthyl)-8H-pyrido[4',3':4,5]thiéno[2,3-e]-1,4-diazépine-8-carboxylate de t-butyle

On ajoute 1 g de 5-(2-chlorophényl)-1,2,3,6,7,9-hexahydro-3-hydroxy-2-oxo-1-(2-phényléthyl)-8H-pyrido[4',3':4,5]thiéno[2,3-e]-1,4-diazépine-8-carboxylate de t-butyle (1,8 mmol) dans un mélange contenant l'acide 7-[(1,1-diméthyléthoxy)carbonyl]amino-heptanoïque (0,45 g, 1,8 mmol), du chlorhydrate de 1-(3-diméthylaminopropyl)-3-éthylcarbodiimide (0,42 g, 2,16 mmol), de la triéthylamine (0,25 ml, 2,2 mmol) et de la 4-diméthylaminopyridine (0,22 g, 1,8 mmol) dans du dichlorométhane (10 ml). On agite 24 h à 23° C puis on ajoute du dichlorométhane (30 ml) et de l'eau glacée (20 ml). On agite et laisse décanter. La phase organique est lavée avec de l'eau (2 x 20 ml), séchée sur sulfate de magnésium. Après évaporation du solvant au rotavapor, on chromatographie sur colonne de silice (éluant : acétate d'éthyle-heptane ; 2-98 à 5-95). On évapore les fractions contenant seulement le produit. On obtient le produit souhaité sous forme d'une poudre amorphe blanche (1,0 g, 71 %). Point de fusion 90-94° C.
RMN$^1$H (400 MHz, $CDCl_3$, δ) : 1,24-1,62 (m, 27H) ; 1,95 (d, 1H) ; 2,5 (m, 2H) ; 2,8-2,9 (m, 4H) ; 3 (m, 1H) ; 3,82 (m, 1H) ; 3,86 (m, 1H) ; 4,35 (m, 2H) ; 4,70 (d, 1H)5,94 (s, 1H) ; 6,73 (m, 1H) ; 7,2-7,3 (m, 5H) ; 7,47-7,56 (m, 4H)
IR ($cm^{-1}$) : 3381 ; 2932 ; 1744 (ν C=O ester) ; 1704-1677 (ν C=O carbamates et ν C=O lactame) ; 1165
HPLC MS : 99,1 % à 250 nm (MH+ trouvée 779,3 ; MH+ théorie 779,32)

2ème étape

3-(7-amino-1-oxo-heptyloxy)-5-(2-chlorophényl)-1,2,3,6,7,9-hexahydro-1-(2-phényléthyl)-8H-pyrido[4',3':4,5]thiéno[2,3-e]-1,4-diazépine-2-one

Le composé obtenu à l'étape précédente (0,95 g, 1,2 mmol) est solubilisé dans du dichlorométhane (2 ml). On ajoute de l'acide trifluoroacétique (1 ml) et on agite 16 h à 23° C. Après évaporation du solvant au rotavapor en s'aidant avec du toluène (2 x 20 ml), on purifie par chromatographie sur colonne de silice (éluant : dichlorométhane-méthanol-triéthylamine 94-6-0 à 89-10-1). Après évaporation des fractions contenant seulement le produit, on obtient le produit souhaité sous forme d'une poudre blanche (0,13 g, 19 %).

RMN$^1$H (400 MHz, $CDCl_3$, δ) : 1,28-1,64 (m, 13H) ; 2-2,2 (m, 1H) ; 2,52 (m, 2H) ; 2,73-2,87 (m, 4H) ; 3,2-3,4 (m, 1H) ; 3,82 (m, 1H) ; 4,26-4,37 (m, 2H) ; 5,94 (s, 1H) ; 7,22-7,55 (m, 9H) ; 7,95 (sé, 2H)
HPLC MS : 96,5 % à 250 nm (MH+ trouvée 579,2 ; MH+ théorie 579,22)

**Exemple 8**

3-(7-amino-1-oxo-heptyloxy)-5-(2-chlorophényl)-1,2,3,6,7,9-hexahydro-N-(4-méthoxy-2-nitrophényl)-2-oxo-1-(2-phényléthyl)-8H-pyrido[4',3':4,5]thiéno[2,3-e]-1,4-diazépine-8-carbothioamide

On solubilise la 3-(7-amine-1-oxo-heptyloxy)-5-(2-chlorophényl)-1,2,3,6,7,9-hexahydro-1-(2-phényléthyl)-8H-pyrido[4',3':4,5]thiéno[2,3-e]-1,4-diazépine-2-one (0,11 g, 0,2 mmol) dans du dichlorométhane (2 ml). On ajoute le 4-méthoxy-2-nitrophénylisothiocyanate (0,047 g, 0,22 mmol). On agite 24 h à 23° C puis on ajoute de l'éther isopropylique (20 ml) jusqu'à précipitation du solide. On agite le mélange hétérogène en rajoutant de l'éther (2 ml) et du dichlorométhane (0,5 ml). Après filtration sur fritté et lavage à l'éther isopropylique, on obtient le produit souhaité sous forme d'une poudre orange (0,045 g, 29 %).
Point de fusion 90-95° C.
HPLC MS : 89,3 % à 250 nm (MH+ trouvée 789,2 ; MH+ théorie 789,23)

**Exemple 9**

4-(7-amino-1-oxo-heptyl)-5-(2-chlorophényl)-1,2,3,4,5,6,7,9-octahydro-1-(2-phényléthyl)-8H-pyrido[4',3':4,5]thiéno[2,3-e]-1,4-diazepine-2-one

<u>1ère étape</u>

5-(2-chlorophényl)-1,2,3,4,5,6,7,9-octahydro-2-oxo-1-(2-phényléthyl)-8H-pyrido[4',3':4,5] thiéno[2,3-e]-1,4-diazépine-8-carboxylate de t-butyle

A température ambiante et sous argon, on ajoute de l'acide acétique (12 ml) à 4 g de 5-(2-chlorophényl)-1,2,3,6,7,9-hexahydro-2-oxo-1-(2-phényléthyl)-8H-pyrido [4',3':4,5] thiéno [2,3-e]-1,4-diazépine-8-carboxylate de t-butyle (7,9 mmol). On ajoute ensuite de l'éthanol (60 ml) puis on refroidit à 0° C et on ajoute lentement du cyanoborohydrure de sodium (1,5 g, 2,4 mmol). Le mélange réactionnel est agité une heure à 23° C puis versé dans l'eau puis on extrait avec du dichlorométhane (2 x 100 ml). La phase organique est lavée avec une solution d'ammoniaque à 10 % (2 x 50 ml), séchée sur sulfate de magnésium. Après filtration et évaporation du solvant, on obtient le produit souhaité sous forme d'un solide blanc amorphe (2,96 g, 75 %).
RMN$^1$H (400 MHz, $CDCl_3$, δ) : 1,36 (s, 9H) ; 1,65 (m, 1H) ; 2,16 (m, 1H) ; 2,78 (m, 1H) ; 2,91 (m, 1H) ; 3,38 (m, 1H) ; 3,42 (m, 2H) ; 3,50 (m, 2H) ; 4,20 (m, 1H) ; 4,40 (m, 1H) ; 4,50 (m, 1H) ; 5,46 (m, 1H) ; 7,30 (m, 8H) ; 7,43 (m, 1H)
IR ($cm^{-1}$) : 3350 (ν NH diazépine) ; 2950 ; 1687 (ν C=O carbamate) ; 1672 (ν C=O lactame) ; 1365 ; 1172 ; 751
HPLC (UV) : 99,4 %

<u>2ème étape</u>

5-(2-chlorophényl)-4-[7-[(1,1-diméthyléthoxycarbonyl)amino]-1-oxo-heptyl]-1,2,3,4,5,6,7,9-octahydro-2-oxo-1-(2-phényléthyl)-8H-pyrido[4',3':4,5]thieno[2,3-e]-1,4-diazépine-8-carboxylate de t-butyle

On solubilise le produit obtenu lors de l'étape précédente (1 g, 1,86 mmol) dans du 1,2-dichloro-éthane (20 ml). On ajoute de la résine N-cyclohexyl carbodiimide, du N'-méthyl polystyrène HI (2,3 g, 5,6 mmol) et du tétrahydrofurane (0,5 ml). On ajoute l'acide 7-[(1,1-diméthyléthoxy)carbonyl]amino-heptanoïque (0,91 g, 3,7 mmol) et on agite 48 h à 23° C. La résine est filtrée. On évapore le solvant et on reprend le milieu réactionnel dans du dichlorométhane (30 ml). On ajoute de la résine basique échangeuse d'ion puis on agite durant 1 heure et on filtre. Après évaporation du solvant au rotavapor, on obtient le produit souhaité sous forme d'un solide blanc amorphe (1,2 g, 86 %).
RMN$^1$H (400 MHz, $CDCl_3$, δ) : 1,15 (m) ; 1,25(m) ; 1,36 (s) ; 1,41 (s) ; 1,52 (m) ; 2,85 (m) ; 2,16 (m) ; 2,22-2,40 (m) ; 2,58 (m) ; 2,81 (m) ; 3,48 (m) ; 3,75 (m) ; 3,90 (m) ; 4,35 (m) ; 4,40-4,60 (m) ; 6,0-7,0 (m) ; 7,30 (m) ; 7,18-7,40 (m)
IR ($cm^{-1}$) : 3378 (ν NH carbamate) ; 2974 ; 2930 ; 1700 (ν C=O carbamates) ; 1676 (ν C=O lactame);1391 ; 1168 ; 759

<u>3ème étape</u>

4-(7-amino-1-oxo-heptyl)-5-(2-chlorophényl)-1,2,3,4,5,6,7,9-octahydro-1-(2-phényléthyl)-8H-pyrido[4',3':4,5]thiéno[2,3-e]-1,4-diazepine-2-one

On agite 4 h à 23° C une solution contenant le composé obtenu lors de l'étape précédente (1 g, 1,3 mmol) et de l'acide trifluoroacétique (3 ml) dans 10 ml de dichlorométhane. On évapore le mélange réactionnel en reprenant

avec du toluène (2 x 30 ml) et de l'éther (2 x 30 ml). On agite le résidu dans un mélange de solvant contenant éther-acétone-dichlorométhane (90-0,5-0,5) pendant 5 minutes. On filtre sur fritté puis on lave à l'éther. On obtient le produit souhaité sous forme d'une poudre blanche (1,0 g, 96 %).
Point de fusion : 149-152° C
RMN$^1$H (400 MHz, $CDCl_3$, δ) : 1,20-1,57 (m) ; 1,86-2,25 (m) ; 2,56-2,79 (m) ; 2,91 (m) ; 3,30 (m) ; 3,79-3,91 (m) ; 4,27-4,37 (m) ; 6,07-6,63 (m) ; 6,63-6,99 (m) ; 7,01-7,76 (m) ; 9,45 (m).

**Exemple 10**

4-(7-amino-1-oxo-heptyl)-5-(2-chlorophényl)-1,2,3,4,5,6,7,9-octahydro-N-(4-méthoxy-2-nitrophényl)-2-oxo-1-(2-phényléthyl)-8H-pyrido[4',3':4,5]thieno[2,3-e]-1,4-diazépine-8-carbothioamide

On ajoute de la triéthylamine (0,22 ml, 1,5 mmol) à 0,5 g de 4-(7-amino-1-oxo-heptyl)-5-(2-chlorophényl)-1,2,3,4,5,6,7,9-octahydro-1-(2-phényléthyl)-8H-pyrido[4',3':4,5]thieno [2,3-e] -1,4-diazépine-2-one (0,63 mmol) dissout dans du dichlorométhane (12 ml). On ajoute ensuite du 4-méthoxy-2-nitro-phénylisothiocyanate (0,146 g, 0,69 mmol) et on agite une heure à 23° C puis on évapore le solvant au rotavapor. Après purification par chromatographie sur colonne de silice (éluant : dichlorométhane-méthanol-amoniaque : 98-2-0 à 95-5-0,5), on évapore les fractions contenant seulement le produit puis on ajoute un minimum de dichlorométhane puis une solution d'acide chlorhydrique 1M (0,2 ml). On agite à 23° C pendant 30 minutes puis on filtre sur fritté le produit obtenu. Après lavage à l'éther, on obtient le produit souhaité sous forme d'une poudre orange (0,12 g, 25 %).
Point de fusion : à partir de 160° C
RMN$^1$H (400 MHz, $CDCl_3$, δ) : 1,32 (m) ; 1,57 (m) ; 1,9 (m) ; 2,10 (m) ; 2,33 (m) ; 2,43-2,75 (m) ; 3,57-3,35 (m) ; 3,83 (m) ; 4,35 (m) ; 5,00 (m) ; 5,30 (m) ; 6,65 (m) ; 7,00 (m) ; 7,50-7,01 (m) ; 7,89 (m) ; 9,69 (m)
IR ($cm^{-1}$) : 3420 (ν NH) ; 2930 ; 1670 ; 1664 ; 1655 ; 1649 ; 1532 ; 1348
HPLC (UV) : 96 %

**Exemple 11**

5-(2-chlorophényl)-1,2,3,4,5,6,7,9-octahydro-4-(3-méthyl-2-butenyl)-2-oxo-1-(2-phényl-éthyl)-8H-pyrido[4', 3':4,5]thiéno[2,3-e]-1,4-diazepine

<u>1$^{ère}$ étape</u>

5-(2-chlorophényl)-1,2,3,4,5,6,7,9-octahydro-4-(3-méthyl-2-butenyl)-2-oxo-1-(2-phényl-éthyl)-8H-pyrido[4', 3':4,5]thiéno[2,3-e]-1,4-diazépine-8-carboxylate de t-butyle

On dissout 0,5 g de 5-(2-chlorophényl)-1,2,3,4,5,6,7,9-octahydro-2-oxo-1-(2-phényléthyl)-8H-pyrido[4',3':4,5] thiéno[2,3-e]-1,4-diazépine-8-carboxylate de t-butyle (1 mmol) dans du tétrahydrofurane anhydre (20 ml). On ajoute par portion l'hydrure de sodium dispersé à 60 % (0,028 g, 1,1 mmol). On attend la fin du dégagement puis on ajoute goutte à goutte le 1-bromo-3-méthyl-2-butène (0,125 ml, 1,1 mmol). On agite 3 h à 23° C et on chauffe toute la nuit à 60° C puis on rajoute du dérivé halogéné (0,125 ml, 1,1 mmol). On chauffe 48 h à 60° C. Le milieu réactionnel est versé dans une solution saturée en chlorure d'ammonium (30 ml) puis on ajoute de l'acétate d'éthyle (30 ml). Après décantation, la phase aqueuse est extraite avec de l'acétate d'éthyle (30 ml). La phase organique est séchée sur sulfate de magnésium, filtrée et le solvant évaporé. Après purification par chromatographie sur colonne de silice (éluant : acétate d'éthyle-heptane : 20-80 à 50-50), les fractions contenant seulement le produit sont évaporées et on obtient le produit souhaité sous forme d'une poudre amorphe jaune pâle (0,47 g, 79 %).
RMN$^1$H (400 MHz, $CDCl_3$, δ) : 1,37 (s, 9H) ; 1,53 (s, 3H) ; 1,67 (s, 3H) ; 2,06-2,48 (m, 2H) ; 2,49 (m, 1H) ; 2,61 (m, 1H) ; 3,11 (m, 2H) ; 3,23 (m, 1H) ; 3,3 (m, 1H) ; 3,48 (m, 2H) ; 3,70 (m, 1H) ; 4,46 (m, 1H) ; 4,46 (m, 2H) ; 5,17 (m, 2H) ; 5,17 (m, 2H) ; 7,17-7,55 (m, 9H).
IR ($cm^{-1}$) : 2974 ; 2926 ; 2855 ; 1697 (ν C=O carbamate) ; 1676 (ν C=O lactame) ; 1365 ; 1165 ; 750 ; 699
HPLC (UV) : 98,5 %

<u>2$^{ème}$ étape</u>

5-(2-chlorophényl)-1,2,3,4,5,6,7,9-octahydro-4-(3-méthyl-2-butenyl)-1-(2-phényléthyl)-8H-pyrido[4',3':4,5] thiéno[2,3-e]-1,4-diazépine-2-one

On dissout le composé obtenu lors de l'étape précédente (0,4 g, 0,65 mmol) dans du dichlorométhane (5 ml) puis on ajoute de l'acide trifluoroacétique (2 ml). On laisse agiter durant 2 h à 23° C puis on évapore le solvant et l'excès d'acide au rotavapor. Après purification par chromatographie sur colonne de silice (éluant : dichlorométhane-méthanol-amoniaque : 98-0-0 à 98-2-0,2), on évapore les fractions contenant seulement le produit. On reprend l'huile

résiduelle dans de l'éther (20 ml). Après refroidissement, on ajoute de l'éther chlorhydrique 1 M (0,2 ml) et on agite 15 minutes le solide obtenu qui est ensuite filtré sur fritté et lavé à l'éther. On obtient le produit souhaité sous forme d'une poudre jaune pâle (0,11 g, 33 %).
Point de fusion : 114-118° C
RMN$^1$H (400MHz, $CDCl_3$, δ) : 1,55 (s, 3H) ; 1,69 (s, 3H) ; 2,3-2,65 (m, 4H) ; 3,14-3,40 (m, 7H) ; 3,68 (m, 1H) ; 3,97 (m, 1H) ; 4,27 (m, 2H) ; 5,14 (m, 2H) ; 7,16-7,56 (m, 9H) ; 9,50 (m, 2H).

**Exemple 12**

5-(2-chlorophényl)-1,2,3,4,5,6,7,9-octahydro-4-(3-méthyl-2-butenyl)-N-(4-méthoxy-2-nitrophényl)-2-oxo-1-(2-phényléthyl)-8H-pyrido[4',3':4,5]thiéno[2,3-e]-1,4-diazépine-8-carbothioamide

On solubilise 0,22 g de 5-(2-chlorophényl)-1,2,3,4,5,6,7,9-octahydro-4-(3-méthyl-2-butenyl)-1-(2-phényléthyl)-8H-pyrido[4',3':4,5]thiéno[2,3-e]-1,4-diazépine-2-one (0,43 mmol) dans du dichlorométhane (2 ml) puis on ajoute le 4-méthoxy-2-nitro-phénylisothiocyanate (0,1 g, 0,48 mmol). Après agitation pendant 1 h à 23° C, le solvant est évaporé. Après purification par chromatographie sur colonne de silice (éluant : dichlorométhane-méthanol : 100-0 à 99,5-0.5), les fractions contenant seulement le produit sont évaporées et le solide obtenu repris dans de l'isopentane (20 ml). On ajoute de l'éther (2 ml) puis on agite durant 20 minutes, filtre sur fritté et lave avec un minimun d'éther isopropylique et de l'isopentane. On obtient le produit souhaité sous forme d'une poudre jaune pâle (0,19 g, 61 %).
Point de fusion : 80-84° C
RMN$^1$H (400 MHz, $CDCl_3$, δ) : 1,55 (s, 3H) ; 1,69 (s, 3H) ; 2,25-2,67 (m, 3H) ; 3.07-3,26 (m, 4H) ; 3,70 (m, 1H) ; 3,84 (s, 3H) ; 3,96 (m, 2H) ; 4,10 (m, 1H) ; 5.00-5.18 (m, 4H) ; 6,86-7,59 (m, 13H) ; 9,52 (s, 1H).
IR ($cm^{-1}$) : 3482 ; 2974 ; 2926 ; 2855 ; 1671 (ν C=O lactame) ; 1500 ; 1302
HPLC (UV) : 98 %

**Exemple 13**

5-(2-chlorophényl)-1,3,4,5,6,7,8,9-octahydro-1-(2-phényléthyl)-2H-pyrido[4',3':4,5] thieno[2,3-e]-1,4-diazépine-2-one

On solubilise le 5-(2-chlorophényl)-1,2,3,4,5,6,7,9-octahydro-2-oxo-1-(2-phényléthyl)-8H-pyrido[4',3';4,5] thiéno[2,3-e]-1,4-diazépine-8-carboxylate de t-butyle (0,5 g, 0,93 mmol) dans du dichlorométhane anhydre (10 ml). On ajoute l'acide trifluoroacétique (3 ml) et on agite à une température voisine de 20° C pendant deux heures. On évapore les solvants en reprenant plusieurs fois avec du toluène. Un mélange de solvant (éther-dichlorométhane-acétone 30-0,5-0,5 ml) est ajouté puis le mélange réactionnel est agité deux heures jusqu'à précipitation. Après filtration sur fritté, lavage à l'éther puis séchage sous vide, on obtient le produit souhaité sous forme d'une poudre jaune (0,34 g, 85 %)
Point de fusion : 128-130° C
HPLC MS : 97 % à 270 nm (MH+ trouvée 438,1 ; MH+ théorie 438,14)

**Exemple 14**

5-(2-chlorophényl)-1,2,3,4,5,6,7,9-octahydro-N-(4-méthoxy-2-nitro-phényl)-2-oxo-1-(2-pentyl)-8H-pyrido[4', 3':4,5]thiéno[2,3-e]-1,4-diazépine-8-carbothioamide

On ajoute de l'acide acétique (0,5 ml) sur 0,16 g de 5-(2-chlorophényl)-1,2,3,6,7,9-hexahydro-N-(4-méthoxy-2-nitrophényl)-2-oxo-1-(2-pentyl)-8H-pyrido[4',3':4,5] thieno [2,3-e]-1,4-diazépine-8-carbothioamide (0,26 mmol). On ajoute à cette suspension jaune de l'éthanol (3,5 ml) puis on rajoute goutte à goutte de l'acide acétique (1 ml) et enfin du cyanoborohydrure de sodium (0,033 g, 0,52 mmol). Le mélange réactionnel est agité pendant une heure puis on chauffe une heure à 40° C jusqu'à solubilisation. On agite ensuite 2 heures à 22° C. Le milieu réactionnel est versé dans de l'eau glacée (30 ml). On agite, filtre puis lave abondamment à l'eau. On sèche sous vide sans chauffer et on obtient le produit souhaité sous forme d'une poudre orange (0,145 g, 83 %)
Point de fusion : à partir de 90° C
HPLC MS : 94 % à 240 nm (M trouvée 614,1 ; MH+ théorie 614,17)

D'autres composés, obtenus selon des procédés analogues à ceux décrits dans les exemples 1 à 14, sont présentés ci-dessous. Les exemples 15 à 45 illustrent les composés dans lesquels A---B représente -C=N- ; les exemples 46 à 70 illustrent les composés dans lesquels A---B représente -C-N($R_5$)-.

Les radicaux $R_1$ ; $R'_1$ ; Y ; $R_2$ ; $R_{3a}$ ; $R_{3b}$ ; X ; n ; $R'_4$ ; $R''_4$ ainsi que le point de fusion (en °C) complété éventuellement par l'état du composé (bl : base libre ; st : sel (TFA) ; sh : sel (HCl) ; * état amorphe) des exemples 15 à 45 ont respectivement les valeurs suivantes :

ex 15 : H ; - ; - ; 2-Cl-Phe ; H ; H ; O; 2 ; H ; Phe ; 84-88 (* bl) ;

ex 16 : H ; - ; - ; 2-Cl-Phe ; H ; H ; O ; 0 ; H ; phénylcarbonyle ; 132-134 (bl) ;

ex 17 : H ; - ; - ; 2-Cl-Phe ; H ; H ; O ; 0 ; H ; Phe ; 135-136 (bl) ;

ex 18 : H ; - ; - ; 2-Cl-Phe ; H ; H ; O ; 0 ; H ; cyclohexyle ; 189-190 (bl) ;

ex 19 : H ; - ; - ; 2-Cl-Phe ; H ; H ; O ; 4 ; H ; H ; 144-150 (bl) ;

ex 20 : H ; - ; - ; 2-Cl-Phe ; H ; H ; O ; 2 ; Phe ; Phe ; 104-108 (bl) ;

ex 21 : H ; - ; - ; 2-Cl-Phe ; H ; H ; O ; 2 ; Me ; Me ; 74-77 (* bl) ;

ex 22 : H ; - ; - ; 2-Cl-Phe ; H ; H ; O ; 0 ; H ; adamantyle ; 222-230 (sh) ;

ex 23 : H ; - ; - ; 2-Cl-Phe ; H ; H ; O ; 1 ; H ; pyridyle ; 82 (* bl) ;

ex 24 : H ; - ; - ; Phe ; H ; H ; O ; 1 ; H ; Phe ; >80 (* bl) ;

ex 25 : H ; - ; - ; 4-Cl-Phe ; H ; H ; O ; 1 ; H ; Phe ;118-120 (bl) ;

ex 26 : H ; - ; - ; 2-F-Phe ; H ; H ; O ; 1 ; H ; Phe ; >85 (* bl)

ex 27 : H ; - ; - ; 4-F-Phe ; H ; H ; O ; 1 ; H ; Phe ; >70 (* bl)

ex 28 : H ; - ; - ; 2-Me-Phe ; H ; H ; O ; 1 ; H ; Phe ; 80-82 (* bl) ;

ex 29 : H ; - ; - ; t-butyle ; H ; H ; O ; 1 ; H ; Phe ; 160-162 (sh) ;

ex 30 : $R'_1$-NH-C(Y)- ; 2-$NO_2$-4-MeO-Phe ; S ; 2-Cl-Phe ; H ; H ; O ; 2 ; H ; Phe ; 141-143 ;

ex 31 : $R'_1$-NH-C(Y)- ; 2-$NO_2$-4-MeO-Phe; S ; 2-Cl-Phe ; H ; H ; O ; 0 ; H ; phénylcarbonyle ; 182-184 ;

ex 32 : $R'_1$-NH-C(Y)- ; 2-$NO_2$-4-MeO-Phe ; S ; 2-Cl-Phe ; H ; H ; O ; 0 ; H ; Phe ; 190-192 ;

ex 33 : $R'_1$-NH-C(Y)- ; 2-$NO_2$-4-MeO-Phe ; S ; 2-Cl-Phe ; H ; H ; O ; 4 ; H ; H ; 158-160 ;

ex 34 : $R'_1$-NH-C(Y)- ; 2-$NO_2$-4-MeO-Phe ; S ; 2-Cl-Phe ; H ; H ; O ; 0 ; H ; cyclohexyle ; 183-184 ;

ex 35 : $R'_1$-NH-C(Y)- ; 2-$NO_2$-4-MeO-Phe ; S ; 2-Cl-Phe ; H ; H ; O ; 2 ; Phe ; Phe ; 156-158 ;

ex 36 : $R'_1$-NH-C(Y)- ; 2-$NO_2$-4-MeO-Phe ; S ; 2-Cl-Phe ; H ; H ; O ; 2 ; Me ; Me ; 149-154 ;

ex 37: $R'_1$-NH-C(Y)- ; 2-$NO_2$-4-MeO-Phe ; S ; 2-Cl-Phe ; H ; H ; O ; 0 ; H ; adamantyle ; 210-214 ;

ex 38 : $R'_1$-NH-C(Y)- ; 2-$NO_2$-4-MeO-Phe ; S ; 2-Cl-Phe ; H ; H ; O ; 1 ; H ; pyridyle ; 131 ;

ex 39 : $R'_1$-NH-C(Y)- ; 2-$NO_2$-4-MeO-Phe ; S ; Phe ; H ; H ; O ; 1 ; H ; Phe ; 148-150 ;

ex 40 : $R'_1$-NH-C(Y)- ; 2-$NO_2$-4-MeO-Phe ; S ; 4-Cl-Phe ; H ; H ; O ; 1 ; H ; Phe ; > 130 (*)

ex 41 : $R'_1$-NH-C(Y)- ; 2-$NO_2$-4-MeO-Phe ; S ; 2-F-Phe ; H ; H ; O ; 1 ; H ; Phe ; > 122 (*) ;

ex 42 : $R'_1$-NH-C(Y)- ; 2-$NO_2$-4-MeO-Phe ; S ; 4-F-Phe ; H ; H ; O ; 1 ; H ; Phe ; 108-110 ;

ex 43 : $R'_1$-NH-C(Y)- ; 2-$F_3C$-Phe ; O ; 4-F-Phe ; H ; H ; O ; 1 ; H ; Phe ; >85 (*) ;

ex 44 : $R'_1$-NH-C(Y)- ; 2-$NO_2$-4-MeO-Phe ; S ; 2-Me-Phe ; H ; H ; O ; 1 ; H ; Phe ; 120-130 ;

ex 45 : $R'_1$-NH-C(Y)- ; 2-$NO_2$-4-MeO-Phe ; S ; t-butyle ; H ; H ; O ; 1 ; H ; Phe ; 177 ;

Les radicaux $R_1$ ; $R'_1$ ; Y ; $R_2$ ; $R_{3a}$ ; $R_{3b}$ ; $R_5$ ; X ; n ; $R'_4$ ; $R''_4$ ainsi que le point de fusion (en °C complété éventuellement par l'état du composé (b1 : base libre ; st : sel (TFA) ; sh : sel (HCl) ; * état amorphe) des exemples 46 à 70 ont respectivement les significations suivantes :

ex 46 : H ; - ; - ; 2-Cl-Phe ; H ; H ; H ; O ; 2 ; Me ; Me ; 128-132 (sh) ;

ex 47 : H ; - ; - ; 2-Cl-Phe ; H ; H ; aminopentylcarbonyl ; O ; 1 ; H ; Phe ; 200-202 (st) ;

ex 48 : H ; - ; - ; 2-Cl-Phe ; H ; H ; indolylméthylcarbonyl ; O ; 1 ; H ; Phe ; 160 (st) ;

ex 49 : H ; - ; - ; 2-Cl-Phe ; H ; H ; aminobutylcarbonyl ; O ; 1 ; H ; Phe ; 120-124 (st) ;

ex 50 : H ; - ; - ; 2-Cl-Phe ; H ; H ; propylcarbonyl ; O ; 1 ; H ; Phe ; 214 (st) ;

ex 51 : H ; - ; - ; 2-Cl-Phe ; H ; H ; cyclopentyl-méthylcarbonyl ; O ; 1 ; H ; Phe ; 175-185 (st) ;

ex 52 : H ; - ; - ; 2-Cl-Phe ; H ; H ; phényl-propylcarbonyl ; O ; 1 ; H ; Phe ; >155 (st) ;

ex 53 : H ; - ; - ; 2-Cl-Phe ; H ; H ; phényléthylcarbonyl ; O ; 1 ; H ; Phe ; 149-151 (st) ;

ex 54 : H ; - ; - ; 2-Cl-Phe ; H ; H ; 4-(L-alanoyloxyméthyl) benzyl carbonyl ; O ; 1 ; H ; Phe ; > 140 (st).

ex 55 : H ; - ; - ; 2-Cl-Phe ; H ; H ; 4-(aminométhyl)phényl carbonyl ; O ; 1 ; H ; Phe (b1).

ex 56 : H ;- ; - ; Phe ; H ; H ; $NH_2$-$CH_2$-C(O)-NH-$CH_2$-C(O)-NH-C(O)- ; O ; 2 ; Me ; Me ; 122-128 (st).

ex 57 : H ; - ; - ; néopentyl ; H ; H ; aminohexylcarbonyl ; O ; 1 ; H ; Phe ; < 50.

ex 58 : H ; - ; - ; isobutyl ; H ; H ; aminohexylcarbonyl ; O ; 1 ; H ; Phe ; à partir de 60° C (*).

ex 59 : H ; - ; - ; isobutyl ; H ; H ; H ; O ; 1 ; H ; Phe ; 202-206 (sh).

ex 60 $R'_1$-NH-C(Y)- ; 2-$NO_2$-4-MeO-Phe ; S ; 2-Cl-Phe ; H ; H ; propylcarbonyl ; O ; 1 ; H ; Phe ; 163-165

ex 61: $R'_1$-NH-C(Y)- ; 2-$NO_2$-4-MeO-Phe ; S ; 2-Cl-Phe ; H ; H ; cyclopentyl-méthylcarbony] ; O ; 1 ; H ; Phe ; 177-178 ;

ex 62 : $R'_1$-NH-C(Y)- ; 2-$NO_2$-4-MeO-Phe ; S ; 2-Cl-Phe ; H ; H ; phényl-propylcarbonyl ; O ; 1 ; H ; Phe ; 202-203 ;

ex 63 : $R'_1$-NH-C(Y)- ; 2-$NO_2$-4-MeO-Phe ; S ; 2-Cl-Phe ; H ; H ; phényléthylcarbonyl ; O ; 1 ; H ; Phe ; 114-115 ;

ex 64 : $R'_1$-NH-C(Y)- ; 2-$NO_2$-4-MeO-Phe ; S ; 2-Cl-Phe ; H ; H ; aminobutylcarbonyl ; O ; 1 ; H ; Phe ; 166-172 (sh) ;

ex 65 : $R'_1$-NH-C(Y)- ; 2-$NO_2$-4-MeO-Phe ; S ; 2-Cl-Phe ; H ; H ; indolylméthylcarbonyl ; O ; 1 ; H ; Phe ; 193-196 ;

ex 66 : $R'_1$-NH-C(Y)- ; 2-$NO_2$-4-MeO-Phe ; S ; 2-Cl-Phe ; H ; H ; aminopentylcarbonyl ; O ; 1 ; H ; Phe ; >150 (sh).

ex 67 : $R'_1$-NH-C(Y)- ; 2-$NO_2$-4-MeO-Phe ; S ; Phe ; H ; H ; $NH_2$-$CH_2$-C(O)-NH-$CH_2$-C(O)-NH-C(O)- ; O ; 2 ; Me ; Me ; 184-188 (sh).

ex 68 : $R'_1$-NH-C(Y)- ; 2-$NO_2$-4-MeO-Phe ; S ; Phe ; H ; H ; aminohexylcarbonyl ; O ; 2 ; Me ; Me ; 160-166 (sh).

ex 69 : $R'_1$-NH-C(Y)- ; 2-$NO_2$-4-MeO-Phe ; S ; néopentyl ; H ; H ; aminohexylcarbonyl ; O ; 1 ; H ; Phe ; 116-118 (bl).

ex 70 : $R'_1$-NH-C(Y)- ; 2-$NO_2$-4-MeO-Phe ; S ; isobutyl; H ; H ; aminohexylcarbonyl ; O ; 1 ; H ; Phe ; à partir de 110°C (bl).

**Etude pharmacologique**

Etude de liaison aux récepteurs de la somatostatine

L'affinité des composés de l'invention sur les récepteurs humains de la somatostatine, est déterminée par la mesure de l'inhibition de la liaison de la somatostatine-14 marquée à l'iode-125 ([125I-Tyr11]SRIF-14) sur les récepteurs de cellules CHO-K11 transfectées.

Les gènes humains codant pour chacun des sous types de récepteurs de la somatostatine, sst1, sst2, sst3, sst4 et sst5, ont été isolés et sous-clonés (*Proc. Natl. Acad. Sci.* USA 1992, 89, 251-255 ; *J. Biol. Chem*. 1992, 267, 20422-20428 ; *Mol. Phannacol*. 1992, 42, 2136-2142 ; *Proc. Natl. Acad. Sci*. USA 1993, 90, 4196-4200 ; *Mol. Pharmacol*. 1994, 46, 291-298). Les vecteurs d'expression ont été construits et des lignées cellulaires clonées ont été obtenues par transfection dans des cellules de mammifères CHO-K1. Le plasmide pRSV-neo a été inclus comme facteur de sélection.

Les cellules CHO-K1 qui expriment de façon stable les récepteurs humains de la somatostatine sont cultivées dans du milieu RPMI 1640 contenant 10 % de sérum de veau foetal et 0,4 mg/ml de généticine. Les cellules sont collectées par de l'EDTA à 0.5 mM et centrifugées à 500 g pendant 5 minutes à 4° C. Le culot est resuspendu dans du Tris 50 mM, pH 7,4 et centrifugé deux fois à 500 g pendant 5 minutes à 4° C. Les cellules sont lysées par sonication puis centrifugées à 39000 g pendant 10 minutes à 4° C. Le culot est resuspendu dans le même tampon et centrifugé à 50000 g pendant 5 minutes à 4° C. Les membranes cellulaires obtenues sont conservées à -80° C jusqu'au jour des expériences.

Les expériences d'inhibition compétitives de la liaison de [125I-Tyr11]SRIF-14 sont conduites en duplicat dans des plaques 96 puits. Les membranes cellulaires à 10 (sst2 et sst5) ou 20 (sst 1, sst3 et sst4) µg de protéines/puits, sont incubées avec [125I-Tyr11]SRIF-14 à 0,05 nM (sst2) ou 0,1 nM ( sst1, sst3, sst4 ou sst5) pendant 50 (sst3), 60 (sst1 et sst2), 70 (sst5) ou 90 (sst4) minutes à 37° C dans du tampon HEPES 50 mM, pH 7,4, BSA 0,2 %, $MgCl_2$ 5 mM, Trasylol 200 KIU/ml, bacitricin 0,02 mg/ml, flurorure de phénylméthylsulphonyl 0,02 mg/ml.

Après la période d'incubation, [125I-Tyr11]SRIF-14 libre ou liée aux récepteurs de la somatostatine est séparée sur une unité de filtration (Filtermate 196, Packard) avec des plaques filtrantes Unifilter GF/C (Packard) prétraitées par du polyéthylènimine à 0,1 %. Après lavage avec de l'HEPES 50 mM, la radioactivité présente sur les filtres est mesurée par un compteur Top Count (Packard).

La liaison spécifique obtenue en soustrayant la liaison non-spécifique (déterminée en présence de 0,1 µM de somatostatine-14) de la liaison totale. Les résultats sont analysés par régression non-linéaire (MDL) et les constantes d'inhibition (Ki) déterminées sont comprises entre 10 et 10000 nM.

**Revendications**

1. Composés de formule générale I

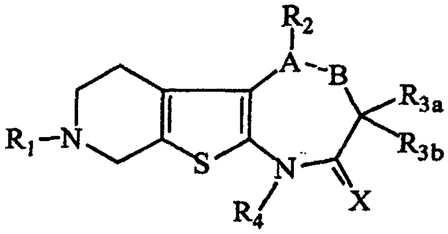

I

sous forme racémique, ou d'énantiomères ou de diastéréoisomères ou toutes combinaisons de ces formes, et dans laquelle

$R_1$ représente l'atome d'hydrogène ou un radical de formule $R'_1$-NH-C(Y)- ;

$R'_1$ représente un radical aryle ou hétéroaryle, les radicaux aryle et hétéroaryle étant éventuellement substitués

par un ou plusieurs substituants, identiques ou différents, choisis parmi les radicaux suivants : alkyle inférieur, alkoxy inférieur, alkylthio inférieur, alkoxy inférieur carbonyle, alkyle inférieur sulfonyle, halo, trifluorométhyle, trifluorométhyloxy, hydroxy, nitro, cyano, aryle, aryloxy, cycloalkyle ou hétérocycloalkyle ;

$R_2$ représente un radical alkyle inférieur, trifluorométhyle ou le radical phényle éventuellement substitué par un ou plusieurs substituants, identiques ou différents, choisis parmi : le radical hydroxy, halo, un radical alkyle inférieur ou alkoxy inférieur ;

X et Y représentent indépendamment O ou S ;

$R_{3a}$ représente l'atome d'hydrogène, un radical alkyle inférieur, hydroxy ou le radical de formule -OC(O) $R'_{3a}$ ;

$R'_{3a}$ représente un radical alkyle comprenant de 1 à 10 atomes de carbone éventuellement substitué par un ou plusieurs substituants, identiques ou différents, choisis parmi les radicaux suivants : cycloalkyle ; hétérocycloalkyle ; aryle ; hétéroaryle ; guanidyle éventuellement substitué par nitro ou cyano ; un radical de formule $NR''_{3a}R'''_{3a}$ dans laquelle $R''_{3a}$ et $R'''_{3a}$ représentent, indépendamment, l'atome d'hydrogène, un radical alkyle inférieur, aryle, arylalkyle inférieur, hétéroarylalkyle inférieur, alkylcarbonyle ou alkoxycarbonyle ;

$R_{3b}$ représente l'atome d'hydrogène ou un radical alkyle inférieur ;

$R_4$ représente un radical de formule $-(CH_2)_n-CHR'_4R''_4$ ;

n représente les valeurs 0, 1, 2, 3, 4, 5 ou 6 ;

$R'_4$ et $R''_4$ représentent, indépendamment, l'atome d'hydrogène, un radical alkyle inférieur, cycloalkyle, cycloalkyle alkyle inférieur, aryle, arylalkyle inférieur, hétéroaryle, hétéroarylalkyle inférieur, arylcarbonyle ou adamantyle, ces radicaux étant éventuellement substitués par un ou plusieurs susbtituants choisis parmi : le radical hydroxy, halo, trifluorométhyle, un radical alkyle inférieur ou alkoxy inférieur ;

A---B représente -C=N- ou $-C-N(R_5)-$ ;

$R_5$ représente l'atome d'hydrogène, un radical alkyle inférieur, alkenyle inférieur ou de formule $-C(O)-(CH_2)_p-R'_5$ ;

$R'_5$ représente l'atome d'hydrogène, un radical amino, alkyl inférieur amino, di(alkyl inférieur)amino, cycloalkyle, hétérocycloalkyle, guanidyle éventuellement substitué par nitro ou cyano, aryle éventuellement substitué par un ou plusieurs substituants identiques ou différents choisis parmi les radicaux alkyle et alkoxyalkyle, les radicaux alkyle et alkoxyalkyle étant eux même éventuellement substitués par les radicaux oxy et amino, hétéroaryle ou un radical de formule $NH-C(O)-(CH_2)_c-NH-C(O)-(CH_2)_d-NH_2$ ;

p représente les valeurs 0, 1, 2, 3, 4, 5, 6, 7, 8, 9 ou 10 ;

c et d représentent indépendamment les valeurs 0, 1, 2 ou 3 ;

ou un sel de ces composés,
étant entendu que le terme "inférieur", lorsqu'il se réfère à un radical alkyle, alkoxy ou alkykthio, signifie un radical comprenant au plus 6 atomes de carbone.

**2.** Composés de formule générale I telle que définie à la revendication 1 et dans laquelle

$R'_1$ représente un radical aryle éventuellement substitué par un ou plusieurs substituants, identiques ou différents, choisis parmi les radicaux suivants: alkoxy inférieur, trifluorométhyle ou nitro ;

$R_2$ représente un radical alkyle inférieur ou le radical phényle éventuellement substitué par un ou plusieurs groupes, identiques ou différents, choisis parmi : halo ou alkyle inférieur ;

$R_{3a}$ représente l'atome d'hydrogène, hydroxy ou le radical de formule -OC(O) $R'_{3a}$ ;

$R'_{3a}$ représente un alkyle, linéaire ou ramifié, comprenant de 1 à 6 atomes de carbone éventuellement substitué par un ou plusieurs substituants, identiques ou différents, de formule $NR''_{3a}R'''_{3a}$ dans laquelle $R''_{3a}$ et $R'''_{3a}$ représentent, indépendamment, l'atome d'hydrogène, un radical alkyle inférieur ou alkoxycarbonyle ;

R3b représente l'atome d'hydrogène ;

$R'_4$ et $R''_4$ représentent, indépendamment, l'atome d'hydrogène, un radical alkyle inférieur, cycloalkyle, aryle, hétéroaryle, arylcarbonyle ou adamantyle ;

A--B représente -C=N.

**3.** Composés de formule générale I telle que définie à la revendication 2 et dans laquelle

$R'_1$ représente un radical phényle éventuellement substitué par un ou plusieurs substituants, identiques ou différents, choisis parmi les radicaux suivants : alkoxy inférieur, trifluorométhyle ou nitro ;

$R_2$ représente un radical alkyle inférieur ou le radical phényle éventuellement substitué par un ou plusieurs groupes, identiques ou différents, choisis parmi : alkyle inférieur, chloro ou fluoro ;

$R'_{3a}$ représente un alkyle, linéaire ou ramifié, comprenant de 1 à 6 atomes de carbone éventuellement substitué par un ou plusieurs groupes amino ;

$R'_4$ et $R''_4$ représentent, indépendamment, l'atome d'hydrogène, alkyle inférieur, cyclohexyle, phényle, pyridyle, phénylcarbonyle ou adamantyle.

**4.** Composés selon l'une des revendications 1 à 3 dans lesquels A---B représente -C=N et les radicaux $R_1$ ; $R'_1$ ; Y ; $R_2$ ; $R_{3a}$ ; $R_{3b}$ ; X ; n ; $R'_4$ ; $R''_4$ ont respectivement les significations suivantes :

H ; - ; - ; 2-Cl-Phe ; H ; H ; O ; 1 ; H ; Phe ;

H ; - ; - ; 2-Cl-Phe ; H ; H ; S ; 1 ; H ; Phe ;

H ; - ; - ; 2-Cl-Phe ; H ; H ; O ; 2 ; H ; Phe ;

H ; - ; - ; 2-Cl-Phe ; H ; H ; O ; 0 ; H ; phénylcarbonyle ;

H ; - ; - ; 2-Cl-Phe ; H ; H ; O ; 0 ; H ; Phe ;

H ; - ; - ; 2-Cl-Phe ; H ; H ; O ; 0 ; H ; cyclohexyle ;

H ; - ; - ; 2-Cl-Phe ; H ; H ; O ; 4 ; H ; H ;

H ; - ; - ; 2-Cl-Phe ; H ; H ; O ; 2 ; Phe ; Phe ;

H ; - ; - ; 2-Cl-Phe ; H ; H ; O ; 2 ; Me ; Me ;

H ; - ; - ; 2-Cl-Phe ; H ; H ; O ; 0 ; H ; adamantyle ;

H ; - ; - ; 2-Cl-Phe ; H ; H ; O ; 1 ; H ; pyridyle ;

H ; - ; - ; Phe ; H ; H ; O ; 1 ; H ; Phe ;

H ; - ; - ; 4-Cl-Phe ; H ; H ; O ; 1 ; H ; Phe ;

H ; - ; - ; 2-F-Phe ; H ; H ; O ; 1 ; H ; Phe ;

H ; - ; - ; 4-F-Phe ; H ; H ; O ; 1 ; H ; Phe ;

H ; - ; - ; 2-Me-Phe ; H ; H ; O ; 1 ; H ; Phe ;

H ; - ; - ; t-butyle ; H ; H ; O ; 1 ; H ; Phe ;

H ; - ; - ; 2-Cl-Phe ; OH ; H ; O ; 1 ; H ; Phe ;

H ; - ; - ; 2-Cl-Phe ; OC(O)-$(CH_2)_6NH_2$ ; H ; O ; 1 ; H ; Phe ;

$R'_1$-NH-C(Y)- ; 2-$NO_2$-4-MeO-Phe ; S ; 2-Cl-Phe ; H ; H ; O ; 1 ; H ; Phe ;

$R'_1$-NH-C(Y)- ; 2-$NO_2$-4-MeO-Phe ; S ; 2-Cl-Phe ; H ; H ; O ; 2 ; H ; Phe ;

$R'_1$-NH-C(Y)- ; 2-$NO_2$-4-MeO-Phe ; S ; 2-Cl-Phe ; H ; H ; S ; 1 ; H ; Phe ;

$R'_1$-NH-C(Y)- ; 2-$NO_2$-4-MeO-Phe; S ; 2-Cl-Phe ; H ; H ; O ; 0 ; H ; phénylcarbonyle ;

$R'_1$-NH-C(Y)- ; 2-$NO_2$-4-MeO-Phe ; S ; 2-Cl-Phe ; H ; H ; O ; 0 ; H ; Phe ;

$R'_1$-NH-C(Y)- ; 2-$NO_2$-4-MeO-Phe ; S ; 2-Cl-Phe ; H ; H ; O ; 4 ; H ; H ;

$R'_1$-NH-C(Y)- ; 2-$NO_2$-4-MeO-Phe ; S ; 2-Cl-Phe ; H ; H ; O ; 0 ; H ; cyclohexyle ;

$R'_1$-NH-C(Y)- ; 2-$NO_2$-4-MeO-Phe ; S ; 2-Cl-Phe ; H ; H ; O ; 2 ; Phe ; Phe ;

$R'_1$-NH-C(Y)- ; 2-$NO_2$-4-MeO-Phe ; S ; 2-Cl-Phe ; H ; H ; O ; 2 ; Me ; Me ;

$R'_1$-NH-C(Y)- ; 2-$NO_2$-4-MeO-Phe ; S ; 2-Cl-Phe ; H ; H ; O ; 0 ; H ; adamantyle ;

$R'_1$-NH-C(Y)- ; 2-$NO_2$-4-MeO-Phe ; S ; 2-Cl-Phe ; H ; H ; O ; 1 ; H ; pyridyle ;

$R'_1$-NH-C(Y)- ; 2-$NO_2$-4-MeO-Phe ; S ; Phe ; H ; H ; O ; 1 ; H ; Phe ;

$R'_1$-NH-C(Y)- ; 2-$NO_2$-4-MeO-Phe ; S ; 4-Cl-Phe ; H ; H ; O ; 1 ; H ; Phe ;

$R'_1$-NH-C(Y)- ; 2-$NO_2$-4-MeO-Phe ; S ; 2-F-Phe ; H ; H ; O ; 1 ; H ; Phe ;

$R'_1$-NH-C(Y)- ; 2-$NO_2$-4-MeO-Phe ; S ; 4-F-Phe ; H ; H ; O ; 1 ; H ; Phe ;

$R'_1$-NH-C(Y)- ; 2-$F_3C$-Phe ; O ; 4-F-Phe ; H ; H ; O ; 1 ; H ; Phe ;

$R'_1$-NH-C(Y)- ; 2-$NO_2$-4-MeO-Phe ; S ; 2-Me-Phe ; H ; H ; O ; 1 ; H ; Phe ;

$R'_1$-NH-C(Y)- ; 2-$NO_2$-4-MeO-Phe ; S ; t-butyle ; H ; H ; O ; 1 ; H ; Phe ;

$R'_1$-NH-C(Y)- ; 2-$NO_2$-4-MeO-Phe ; S ; 2-Cl-Phe ; OH ; H ; O ; 1 ; H ; Phe ;

$R'_1$-NH-C(Y)- ; 2-$NO_2$-4-MeO-Phe ; S ; 2-Cl-Phe ; OC(O)-$(CH_2)_6NH_2$ ; H ; O ; 1 ; H ; Phe ;

$R'_1$-NH-C(Y)- ; 2-$NO_2$-4-MeO-Phe ; S ; 2-Me-Phe ; H ; H ; O ; 1 ; H ; Phe ;

$R'_1$-NH-C(Y)- ; 2-$NO_2$-4-MeO-Phe ; S ; t-butyle ; H ; H ; O ; 1 ; H ; Phe ;

$R'_1$-NH-C(Y)- ; 2-$NO_2$-4-MeO-Phe ; S ; 2-Cl-Phe ; OH ; H ; O ; 1 ; H ; Phe ;

$R'_1$-NH-C(Y)- ; 2-$NO_2$-4-MeO-Phe ; S ; 2-Cl-Phe ; OC(O)-$(CH_2)_6NH_2$ ; H ; O ; 1 ; H ; Phe.

**5.** Composés de formule générale I telle que définie à la revendication 1 et dans laquelle

$R'_1$ représente un radical aryle éventuellement substitué par un ou plusieurs substituants, identiques ou différents, choisis parmi les radicaux suivants : alkoxy inférieur ou nitro ;

$R_2$ représente un radical alkyle inférieur ou phényle éventuellement substitué par un ou plusieurs groupes halo identiques ou différents ;

$R_{3a}$ et $R_{3b}$ représente l'atome d'hydrogène ;

$R'_4$ et $R''_4$ représentent, indépendamment, l'atome d'hydrogène, un radical alkyle inférieur ou aryle ;

A---B représente -C-N($R_5$)- ;

$R_5$ représente l'atome d'hydrogène, un radical alkenyle inférieur ou de formule -C(O)-$(CH_2)_p$-$R'_5$.

**6.** Composés de formule générale I telle que définie à la revendication 5 et dans laquelle

$R'_1$ représente un radical phényle éventuellement substitué par un ou plusieurs substituants, identiques ou différents, choisis parmi les radicaux suivants: alkoxy inférieur ou nitro ;

$R_2$ représente un radical alkyle inférieur ou phényle éventuellement substitué par un groupe chloro ;

$R'_4$ et $R''_4$ représentent, indépendamment, l'atome d'hydrogène, alkyle inférieur ou phényle ;

$R_5$ représente l'atome d'hydrogène, pentenyle ou un radical de formule -C(O)-$(CH_2)_p$-$R'_5$ ;

$R'_5$ représente l'atome d'hydrogène, un radical amino, cyclopentyle, indolyle, de formule -NH-C(O)-$CH_2$-NH-C(O)-$CH_2$-$NH_2$ ou phényle éventuellement substitué par un ou plusieurs substituants, identiques ou différents, choisis parmi les radicaux allyle et alkoxyalkyle, les radicaux alkyle et alkoxyalkyle étant eux même substitués par les radicaux oxy et amino.

**7.** Composés selon l'une des revendications 1, 5 ou 6 dans lesquels A--B représente -C-N($R_5$)- et les radicaux $R_1$ ; $R'_1$ ; Y ; $R_2$ ; $R_{3a}$ ; $R_{3b}$ ; X ; $R_5$ ; n ; $R'_4$ ; $R''_4$ ont respectivement les significations suivantes :

H ; - ; - ; 2-Cl-Phe ; H ; H ; H ; O ; 1 ; H ; Phe ;

H ; - ; - ; 2-Cl-Phe ; H ; H ; H ; O ; 2 ; Me ; Me ;

H ; - ; - ; 2-Cl-Phe ; H ; H ; -$CH_2CH=C(Me)_2$ ; O ; 1 ; H ; Phe ;

H ; - ; - ; 2-Cl-Phe ; H ; H ; aminohexylcarbonyl ; O ; 1 ; H ; Phe ;

H ; - ; - ; 2-Cl-Phe ; H ; H ; aminopentylcarbonyl ; O ; 1 ; H ; Phe ;

H ; - ; - ; 2-Cl-Phe ; H ; H ; indolylméthylcarbonyl ; O ; 1 ; H ; Phe ;

H ; - ; - ; 2-Cl-Phe ; H ; H ; aminobutylcarbonyl ; O ; 1 ; H ; Phe ;

H ; - ; - ; 2-Cl-Phe ; H ; H ; propylcarbonyl ; O ; 1 ; H ; Phe ;

H ; - ; - ; 2-Cl-Phe ; H ; H ; cyclopentyl-méthylcarbonyl ; O ; 1 ; H ; Phe ;

H ; - ; - ; 2-Cl-Phe ; H ; H ; phényl-propylcarbonyl ; O ; 1 ; H ; Phe ;

H ; - ; - ; 2-Cl-Phe ; H ; H ; phényléthylcarbonyl ; O ; 1 ; H ; Phe ;

H ; - ; - ; 2-Cl-Phe ; H ; H ; 4-(L-alanoyloxyméthyl)benzyl carbonyl; O ; 1 ; H ; Phe ;

H ; - ; - ; 2-Cl-Phe ; H ; H ; 4-(aminométhyl)phényl carbonyl ; O ; 1 ; H ; Phe ;

H ; - ; - ; Phe ; H ; H ; $NH_2$-$CH_2$-C(O)-NH-$CH_2$-C(O)-NH-$CH_2$-C(O)- ; O ; 2 ; Me ; Me ;

H ; - ; - ; néopentyl ; H ; H ; aminohexylcarbonyl ; O ; 1 ; H ; Phe ;

H ; - ; - ; isobutyl ; H ; H ; aminohexylcarbonyl ; O ; 1 ; H ; Phe ;

H ; - ; - ; isobutyl ; H ; H ; H ; O ; 1 ; H ; Phe ;

$R'_1$-NH-C(Y)- ; 2-$NO_2$-4-MeO-Phe ; S ; 2-Cl-Phe ; H ; H ; H ; O ; 4 ; H ; H ;

$R'_1$-NH-C(Y)- ; 2-$NO_2$-4-MeO-Phe ; S ; 2-Cl-Phe ; H ; H ; -$CH_2CH$=$C(Me)_2$ ; O ; 1 ; H ; Phe ;

$R'_1$-NH-C(Y)- ; 2-$NO_2$-4-MeO-Phe ; S ; 2-Cl-Phe ; H ; H ; aminohexylcarbonyl ; O ; 1 ; H ; Phe ;

$R'_1$-NH-C(Y)- ; 2-$NO_2$-4-MeO-Phe ; S ; 2-Cl-Phe ; H ; H ; propylcarbonyl ; O ; 1 ; H ; Phe ;

$R'_1$-NH-C(Y)- ; 2-$NO_2$-4-MeO-Phe ; S ; 2-Cl-Phe ; H ; H ; cyclopentyl-méthylcarbonyl ; O ; 1 ; H ; Phe ;

$R'_1$-NH-C(Y)- ; 2-$NO_2$-4-MeO-Phe ; S ; 2-Cl-Phe ; H ; H ; phényl-propylcarbonyl ; O ; 1 ; H ; Phe ;

$R'_1$-NH-C(Y)- ; 2-$NO_2$-4-MeO-Phe ; S ; 2-Cl-Phe ; H ; H ; phényléthylcarbonyl ; O ; 1 ; H ; Phe ;

$R'_1$-NH-C(Y)- ; 2-$NO_2$-4-MeO-Phe ; S ; 2-Cl-Phe ; H ; H ; aminobutylcarbonyl ; O ; 1 ; H ; Phe ;

$R'_1$-NH-C(Y)- ; 2-$NO_2$-4-MeO-Phe ; S ; 2-Cl-Phe ; H ; H ; indolylméthylcarbonyl ; O ; 1 ; H ; Phe ;

$R'_1$-NH-C(Y)- ; 2-$NO_2$-4-MeO-Phe ; S ; 2-Cl-Phe ; H ; H ; aminopentylcarbonyl ; O ; 1 ; H;Phe;

$R'_1$-NH-C(Y)- ; 2-$NO_2$-4-MeO-Phe ; S ; Phe ; H ; H ; $NH_2$-$CH_2$-C(O)-NH-$CH_2$-C(O)-NH-$CH_2$-C(O)- ; O ; 2 ; Me ; Me ;

$R'_1$-NH-C(Y)- ; 2-$NO_2$-4-MeO-Phe ; S ; Phe ; H ; H ; aminohexylcarbonyl ; O ; 2 ; Me ; Me;

$R'_1$-NH-C(Y)- ; 2-$NO_2$-4-MeO-Phe ; S ; néopentyl ; H ; H ; aminohexylcarbonyl ; O ; 1 ; H ; Phe ;

$R'_1$-NH-C(Y)- ; 2-$NO_2$-4-MeO-Phe ; S ; isobutyl ; H ; H ; aminohexylcarbonyl ; O ; 1 ; H ; Phe.

**8.** Procédé de préparation de composés de formule générale I telle que définie à la revendication 1 et dans laquelle A---B représente C=N, $R_1$ l'atome d'hydrogène et $R_{3a}$ l'atome d'hydrogène ou un radical alkyle, procédé qui consiste à faire réagir un composé de formule (I)

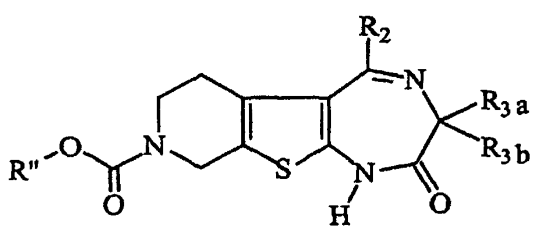

(1)

dans laquelle $R_2$ et $R_{3b}$ ont la signification indiquée à la revendication 1, $R_{3a}$ a la signification indiquée ci-dessus et R" représente un radical alkyle inférieur ou arylalkyle inférieur, avec un composé $R_4Z$ dans laquelle $R_4$ a la signification indiquée à la revendication 1 et Z représente un groupe partant, en présence d'une base forte, pour obtenir le composé (2)

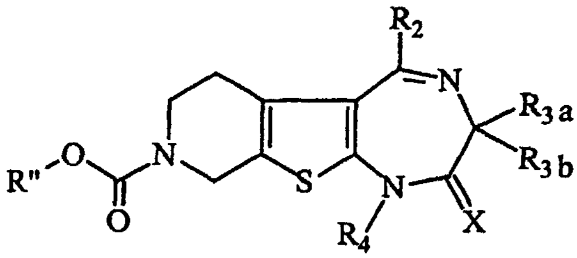

(2)

dans lequel X représente l'atome d'oxygène, composé (2) ainsi obtenu que l'on peut faire réagir avec un réactif de thiation pour obtenir le composé correspondant dans lequel X représente un atome de soufre, composé (2) dans lequel X représente un atome d'oxygène ou de soufre, qui est enfin soumis à une réaction de déprotection du carbamate pour obtenir le produit (I) souhaité.

**9.** Procédé de préparation de composés de formule générale I telle que définie à la revendication 1 et dans laquelle A---B représente -C=N-, $R_1$ l'atome d'hydrogène et $R_{3a}$ le radical hydroxy, procédé qui consiste à oxyder un composé de formule (2) tel que défini à la revendication 8, dans un solvant inerte pour obtenir un composé de formule (3)

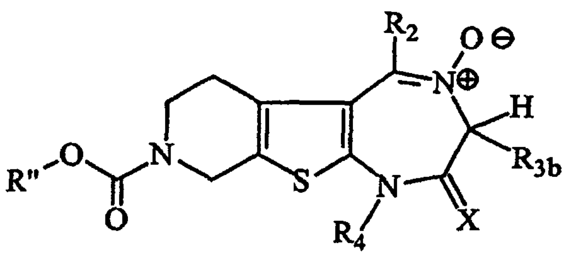

(3)

dans laquelle $R_2$, $R_{3b}$, $R_4$ et X ont la signification indiquée à la revendication 1 et R" a la signification indiquée à la revendication 8, composé de formule (3) que l'on traite avec l'anhydride acétique pour obtenir un composé de formule (4)

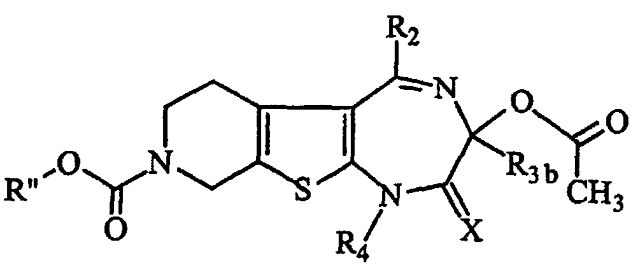

(4)

dans laquelle $R_2$, $R_{3b}$, $R_4$, R" et X ont la signification indiquée ci-dessus, composé (4) qui est ensuite saponifié pour obtenir le composé de formule (5)

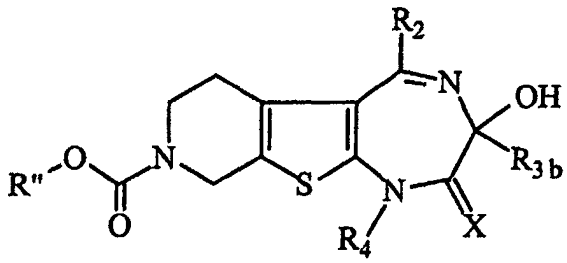

(5)

dans laquelle $R_2$, $R_{3b}$, $R_4$, R" et X ont la signification indiquée ci-dessus,

composé (5) qui est enfin soumis à une réaction de déprotection du carbamate pour obtenir le composé correspondant de formule (I) dans laquelle $R_1$ représente H et $R_{3a}$ le radical hydroxy.

**10.** Procédé de préparation de composés de formule générale I telle que définie à la revendication 1 et dans laquelle A--B représente -C=N-, $R_1$ l'atome d'hydrogène et $R_{3a}$ un radical -OC(O)-$R'_{3a}$ , procédé qui consiste à faire réagir un composé de formule (5) tel que défini à la revendication 9, avec un acide de formule $R'_{3a}$C(O)OH dans laquelle $R'_{3a}$ a la signification indiquée à la revendication 1, pour obtenir un composé de formule (6)

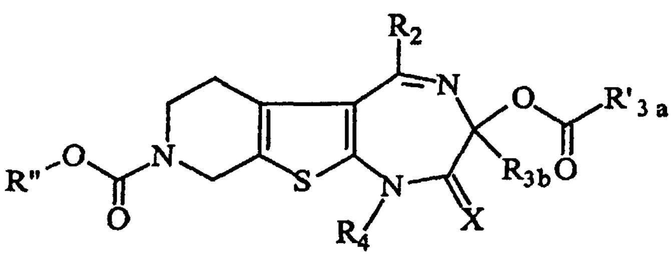

(6)

dans laquelle $R_2$, $R'_{3a}$, $R_{3b}$, $R_4$, R" et X ont la signification indiquée ci-dessus,

composé (6) qui est enfin soumis à une réaction de déprotection du carbamate pour obtenir le composé correspondant de formule (I) dans laquelle $R_1$ représente H et $R_{3a}$ le radical -OC(O)-$R'_{3a}$.

**11.** Procédé de préparation de composés de formule générale I telle que définie à la revendication 1 et dans laquelle A---B représente -C-N($R_5$)-, $R_1$ et $R_5$ un atome d'hydrogène et $R_{3a}$ l'atome d'hydrogène ou un radical alkyle, procédé qui consiste à faire réagir un agent réducteur doux en milieu acide sur un composé de formule (2) tel que défini à la revendication 8, pour obtenir le composé de formule (8)

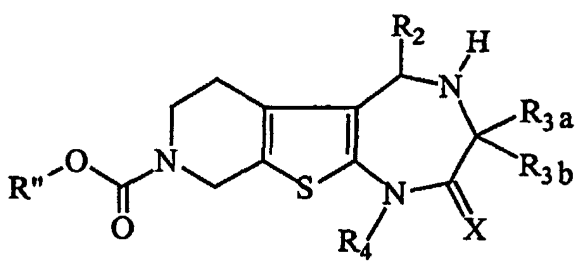

(8)

dans laquelle $R_2$, $R_{3b}$, $R_4$ et X ont la signification indiquée à la revendication 1, $R_{3a}$ a la signification indiquée ci-dessus et R" a la signification indiquée à la revendication 8, composé (8) qui est soumis à une réaction de déprotection du carbamate pour obtenir le produit (I) souhaité dans lequel $R_1$ représente l'atome d'hydrogène.

**12.** Procédé de préparation de composés de formule générale I telle que définie à la revendication 1 et dans laquelle A---B représente -C-N($R_5$)-, $R_1$ représente un atome d'hydrogène, $R_{3a}$ l'atome d'hydrogène ou un radical alkyle

et $R_5$ représente un radical alkenyle,
procédé qui consiste à faire réagir un composé de formule (8) tel que défini à la revendication 11, avec un composé de formule Z'-$R_5$ dans laquelle $R_5$ a la signification indiquée ci-dessus et Z' un groupe partant, en présence d'une base minérale forte dans un solvant inerte, pour obtenir le composé de formule (9)

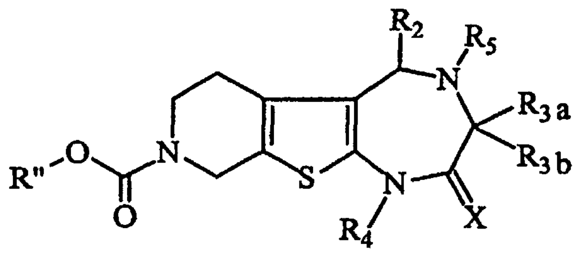

(9)

dans laquelle $R_2$, $R_{3b}$, $R_4$ et X ont la signification indiquée à la revendication 1, $R_{3a}$ et $R_5$ ont la signification indiquée ci-dessus et R" a la signification indiquée à la revendication 8,
composé (9) qui est soumis à une réaction de déprotection du carbamate pour obtenir le produit (I) souhaité dans lequel $R_1$ représente l'atome d'hydrogène.

**13.** Procédé de préparation de composés de formule générale I telle que définie à la revendication 1 et dans laquelle A---B représente -C-N($R_5$)-, $R_1$ représente un atome d'hydrogène, $R_{3a}$ l'atome d'hydrogène ou un radical alkyle et $R_5$ représente un radical-C-$(CH_2)_p$- (O)R'$_5$,
procédé qui consiste à faire réagir un composé de formule (8) tel que défini à la revendication 11, avec un acide de formule R'$_5$-$(CH_2)_p$-C(O)OH dans laquelle R'$_5$ et p ont la signification indiquée ci-dessus, pour obtenir le composé (10) correspondant,

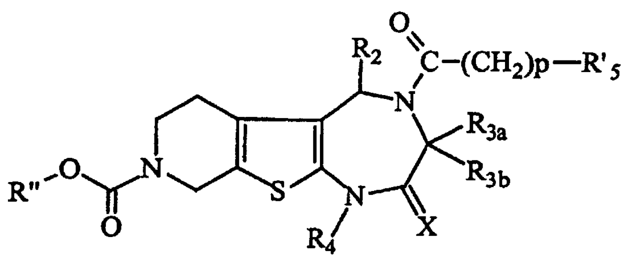

(10)

dans lequel $R_2$, $R_{3b}$, $R_4$, p et X ont la signification indiquée à la revendication 1, $R_{3a}$ et R'$_5$ ont la signification indiquée ci-dessus et R" a la signification indiquée à la revendication 8,
composé (10) qui est soumis à une réaction de déprotection du carbamate pour obtenir le produit (I) souhaité dans lequel $R_1$ représente l'atome d'hydrogène.

**14.** Procédé de préparation de composés de formule générale I telle que définie à la revendication 1 et dans laquelle $R_1$ représente un radical de formule R'$_1$-NH-C(Y)-, procédé qui consiste à faire réagir le composé correspondant de formule (I) dans laquelle $R_1$ représente l'atome d'hydrogène, avec un composé de formule

$$R'_1\text{-N=C=Y} \quad (7)$$

dans laquelle R'$_1$ et Y ont la signification indiquée ci-dessus, pour former le composé de formule I choisi.

**15.** A titre de médicaments, les composés de formule I telle que définie à la revendication 1, ainsi que les sels d'addition avec les acides minéraux ou organiques pharmaceutiquement acceptables desdits produits de formule I.

**16.** A titre de médicaments, les composés de formule I telle que définie à l'une des revendications 2 à 7, ainsi que les

sels d'addition avec les acides minéraux ou organiques pharmaceutiquement acceptables desdits produits de formule I.

**17.** Compositions pharmaceutiques contenant, à titre de principe actif, l'un au moins des médicaments tels que définis à l'une des revendications 15 ou 16, en association avec un support pharmaceutiquement acceptable.

**18.** Utilisation d'un composé selon l'une des revendications 1 à 7, pour la préparation d'un médicament destiné à traiter l'acromégalie, les adénomes hypophysaires et les tumeurs gastroentéropancréatiques endocriniennes.

**19.** Composés de formule (2) tels que définis à la revendication 8.

**Patentansprüche**

**1.** Verbindungen der allgemeinen Formel I

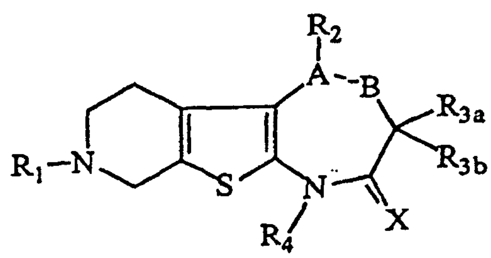

I

in Form von Racemat, von Enantiomeren, von Diastereoisomeren oder in allen Kombinationen dieser Formen, in der

$R_1$ ein Wasserstoffatom oder einen Rest der Formel $R'_1$-NH-C(Y) - darstellt;

$R'_1$ einen Aryl- oder Heteroarylrest darstellt, wobei die Aryl- und Heteroarylreste ggf. durch einen oder,mehrere gleiche oder verschiedene Substituenten substituiert sind, die aus den folgenden Resten ausgewählt sind: Niederalkyl, Niederalkoxy, Niederalkylthio, Niederalkoxycarbonyl, Niederalkylsulfonyl, Halogen, Trifluormethyl, Trifluormethyloxy, Hydroxy, Nitro, Cyano, Aryl, Aryloxy, Cycloalkyl oder Heterocycloalkyl;

$R_2$ einen Niederalkyl-, Trifluormethylrest oder einen Phenylrest, der ggf. durch einen oder mehrere gleiche oder verschiedene Substituenten substituiert ist, die aus den Resten Hydroxy, Halogen, Niederalkyl oder Niederalkoxy ausgewählt sind;

X und Y unabhängig voneinander O oder S darstellen;

$R_{3a}$ ein Wasserstoffatom, einen Niederalkylrest, einen Hydroxyrest oder einen Rest der Formel $-OC(O)R'_{3a}$ darstellt;

$R'_{3a}$ einen Alkylrest mit 1 bis 10 Kohlenstoffatomen darstellt, der ggf. durch einen oder mehrere gleiche oder verschiedene Substituenten substituiert ist, die aus den folgenden Resten ausgewählt sind: Cycloalkyl; Heterocycloalkyl; Aryl; Heteroaryl; ggf. durch Nitro oder Cyano substituiertes Guanidyl; einen Rest der Formel $NR''_{3a}R'''_{3a}$, in der $R''_{3a}$ und $R'''_{3a}$ unabhängig voneinander ein Wasserstoffatom, einen der Reste Niederalkyl, Aryl, Arylniederalkyl, Heteroarylniederalkyl, Alkylcarbonyl oder Alkoxycarbonyl darstellen;

$R_{3b}$ ein Wasserstoffatom oder einen Niederalkylrest darstellt;

$R_4$ einen Rest der Formel $-(CH_2)_n-CHR'_4R''_4$ darstellt;

n die Werte 0, 1, 2, 3, 4, 5 oder 6 darstellt;

$R'_4$ und $R''_4$ unabhängig voneinander ein Wasserstoffätom, einen der Reste Niederalkyl, Cycloalkyl, Cycloalkylniederalkyl, Aryl, Arylniederalkyl, Heteroaryl, Heteroarylniederalkyl, Arylcarbonyl oder Adamantyl darstellen, wobei diese Reste ggf. durch einen oder mehrere Substituenten substituiert sind, die aus den Resten Hydroxy, Halogen, Trifluormethyl, Niederalkyl oder Niederalkoxy ausgewählt sind;

A---B -C=N- oder -C-N($R_5$)- darstellt;

$R_5$ ein Wasserstoffatom, einen Niederalkylrest, einen Niederalkenylrest oder einen Rest der Formel -C(O)-$(CH_2)_p$-$R'_5$ darstellt;

$R'_5$ ein Wasserstoffatom, einen der Reste Amino, Niederalkylamino, Di(niederalkyl)amino, Cycloalkyl, Heterocycloalkyl, Guanidyl, das ggf. durch Nitro oder Cyano substituiert ist, Aryl, das ggf. durch einen oder mehrere gleiche oder verschiedene Substituenten substituiert ist, die aus den Resten Alkyl und Alkoxyalkyl ausgewählt sind, wobei die Reste Alkyl und Alkoxyalkyl ihrerseits ggf. durch die Reste Oxy und Amino substituiert sind, Heteroaryl oder einen Rest der Formel $-NH-C(O)-(CH_2)_c-NH-C(O) - (CH_2)_d-NH_2$ darstellt;

p die Werte 1, 2, 3, 4, 5, 6, 7, 8, 9 oder 10 darstellt;

c und d unabhängig voneinander die Werte 0, 1, 2 oder 3 darstellen;

oder ein Salz dieser Verbindungen,

wobei der Begriff "Nieder", wenn er sich auf einen Alkyl-, Alkoxy- oder Alkylthio-Rest bezieht, einen Rest mit höchstens 6 Kohlenstoffatomen bedeutet.

**2.** Verbindungen der in Anspruch 1 definierten allgemeinen Formel I, in der

$R'_1$ einen Arylrest, der ggf. durch einen oder mehrere gleiche oder verschiedene Substituenten substituiert ist, die aus den folgenden Resten ausgewählt sind: Niederalkoxy, Trifluormethyl oder Nitro;

$R_2$ einen Niederalkylrest oder einen Phenylrest darstellt, der ggf. durch eine oder mehrere gleiche oder verschiedene Gruppen substiuiert ist, die ausgewählt sind aus: Halogen oder Niederalkyl;

$R_{3a}$ ein Wasserstoffatom, Hydroxy oder einen Rest der Formel $-OC(O)R'_{3a}$ darstellt;

$R'_{3a}$ ein verzweigtes oder unverzweigtes Alkyl mit 1 bis 6 Kohlenstoffatomen darstellt, das ggf. durch einen oder mehrere gleiche oder verschiedene Substituenten der Formel $NR''_{3a}R'''_{3a}$ substituiert ist, in der $R''_{3a}$ und $R'''_{3a}$ unabhängig voneinander ein Wasserstoffatom, einen Niederalkylrest oder einen Alkoxycarbonylrest darstellen;

$R_{3b}$ ein Wasserstoffatom darstellt;

$R'_4$ und $R''_4$ unabhängig voneinander ein Wasserstoffatom, einen Rest Niederalkyl, Cycloalkyl, Aryl, Heteroaryl, Arylcarbonyl oder Adamantyl darstellen;

A---B -C=N darstellt.

**3.** Verbindungen der in Anspruch 2 definierten allgemeinen Formel I, in der

$R'_1$ einen Phenylrest darstellt, der ggf. durch einen oder mehrere gleiche oder verschiedene Substituenten substituiert ist, die aus den folgenden Resten ausgewählt sind: Niederalkoxy, Trifluormethyl oder Nitro;

$R_2$ einen Niederalkylrest oder einen Phenylrest darstellt, der ggf. durch eine oder mehrere gleiche oder verschiedene Gruppen substituiert ist, die aus Niederalkyl, Chlor oder Fluor ausgewählt sind;

$R'_{3a}$ ein verzweigtes oder unverzweigtes Alkyl mit 1 bis 6 Kohlenstoffatomen darstellt, das ggf. durch eine oder mehrere Aminogruppen substituiert ist;

$R'_4$ und $R''_4$ unabhängig voneinander ein Wasserstoffatom, ein Niederalkyl, Cyclohexyl, Phenyl, Pyridyl, Phenylcarbonyl oder Adamantyl darstellen.

**4.** Verbindungen nach einem der Ansprüche 1 bis 3, in denen A---B -C=N darstellt und die Reste $R_1$; $R'_1$; Y; $R_2$; $R_{3a}$; $R_{3b}$; X; n; $R'_4$; $R''_4$ jeweils die folgenden Bedeutungen haben:

- H; - ; - ;-2-Cl-Phe; H; H; O; 1; H; Phe;
- H; - ; - ;2-Cl-Phe; H; H; S; 1; H; Phe;
- H; - ; - ;2-Cl-Phe; H; H; O; 2; H; Phe;
- H; - ; - ;2-Cl-Phe; H; H; O; 0; H; Phenylcarbonyl;
- H; - ; - ;2-Cl-Phe; H; H; O; 0; H; Phe;
- H; - ; - ;2-Cl-Phe; H; H; O; 0; H; Cyclohexyl;
- H; - ; -;2-Cl-Phe; H; H; O; 4; H; H;
- H; - ; - ; 2-Cl-Phe; H; H; 0; 2; Phe; Phe;
- H; - ; - ;2-Cl-Phe; H; H; O; 2; Me; Me;
- H; - ; - ;2-Cl-Phe; H; H; O; 0; H; Adamantyl;
- H; - ; - ;2-Cl-Phe; H; H; O; 1; H; Pyridyl;
- H; - ; - ;Phe; H; H; O; 1; H; Phe;
- H; - ; - ;4-Cl-Phe; H; H; O; 1; H; Phe;
- H; - ; - ; -2-F-Phe; H; H; O; 1; H; Phe;
- H; -; -;4-F-Phe; H; H; O; 1; H; Phe;
- H; -; -;2Me-Phe; H; H; O; 1; H; Phe;
- H; -; -;t-Butyl; H; H; O; 1; H; Phe;
- H; -; -;2-Cl-Phe; OH; H; O; 1; H; Phe;
- H; -; -;-2-Cl-Phe; OC(O)-$(CH_2)_6$ $NH_2$; H; O; 1; H; Phe;
- $R'_1$-NH-C(Y)-; 2-$NO_2$-4-MeO-Phe; S; 2-Cl-Phe; H; H; O; 1; H; Phe;
- $R'_1$-NH-C(Y)-; 2-$NO_2$-4-MeO-Phe; S; 2-Cl-Phe; H; H; O; 2; H; Phe;
- $R'_1$-NH-C(Y)-; 2-$NO_2$-4-MeO-Phe; S; 2-Cl-Phe; H; H; S; 1; H; Phe;
- $R'_1$-NH-C(Y)-; 2-$NO_2$-4-MeO-Phe; S; 2-Cl-Phe; H; H; O; 0; H; Phenylcarbonyl;
- $R'_1$-NH-C(Y)-; 2-$NO_2$-4-MeO-Phe; S; 2-Cl-Phe; H; H; O; 0; H; Phe;
- $R'_1$-NH-C(Y)-; 2-$NO_2$-4-MeO-Phe; S; 2-Cl-Phe; H; H; O; 4; H; H;
- $R'_1$-NH-C(Y)-; 2-$NO_2$-4-MeO-Phe; S; 2-Cl-Phe; H; H; O; 0; H; Cyclohexyl;
- $R'_1$-NH-C(Y)-; 2-$NO_2$-4-MeO-Phe; S; 2-Cl-Phe; H; H; O; 2; Phe; Phe;
- $R'_1$-NH-C(Y)-; 2-$NO_2$-4-MeO-Phe; S; 2-Cl-Phe; H; H; O; 2; Me; Me;
- $R'_1$-NH-C(Y)-; 2-$NO_2$-4-MeO-Phe; S; 2-Cl-Phe; H; H; O; 0; H; Adamantyl;
- $R'_1$-NH-C(Y)-; 2-$NO_2$-4-MeO-Phe; S; 2-Cl-Phe; H; H; O; 1; H; Pyridyl;
- $R'_1$-NH-C(Y)-; 2-$NO_2$-4-MeO-Phe; S; Phe; H; H; O; 1; H; Phe;
- $R'_1$-NH-C(Y)-; 2-$NO_2$-4-MeO-Phe; S; 4-Cl-Phe; H; H; O; 1; H; Phe;
- $R'_1$-NH-C(Y)-; 2-$NO_2$-4-MeO-Phe; S; 2-F-Phe; H; H; O; 1; H; Phe;
- $R'_1$-NH-C(Y)-; 2-$NO_2$-4-MeO-Phe; S; 4-F-Phe; H; H; O; 1; H; Phe;
- $R'_1$-NH-C(Y)-; 2-$F_3C$-Phe; O; 4-F-Phe; H; H; O; 1; H; Phe;
- $R'_1$-NH-C(Y)-; 2-$NO_2$-4-MeO-Phe; S; 2-Me-Phe; H; H; O; 1; H; Phe;
- $R'_1$-NH-C(Y)-; 2-$NO_2$-4-MeO-Phe; S; t-Butyl; H; H; O; 1; H; Phe;
- $R'_1$-NH-C(Y)-; 2-$NO_2$-4-MeO-Phe; S; 2-Cl-Phe; OH; H; O; 1; H; Phe;
- $R'_1$-NH-C(Y)-; 2-$NO_2$-4-MeO-Phe; S; 2-Cl-Phe; OC(O)-$(CH_2)_6$ $NH_2$; H; O; 1; H; Phe;
- $R'_1$-NH-C(Y)-; 2-$NO_2$-4-MeO-Phe; S; 2-Me-Phe; H; H; O; 1; H; Phe;
- $R'_1$-NH-C(Y)-; 2-$NO_2$-4-MeO-Phe; S; t-Butyl; H; H; O; 1; H; Phe;
- $R'_1$-NH-C(Y)-; 2-$NO_2$-4-MeO-Phe; S; 2-Cl-Phe; OH; H; O; 1; H; Phe;
- $R'_1$-NH-C(Y)-; 2-$NO_2$-4-MeO-Phe; S; 2-Cl-Phe; OC(O)-$(CH_2)_6NH_2$; H; O; 1; H; Phe.

**5.** Verbindungen der in Anspruch 1 definierten allgemeinen Formel I, in der

$R'_1$ einen Arylrest darstellt, der ggf. durch einen oder mehrere gleiche oder verschiedene Substituenten substituiert ist, die aus den folgenden Resten ausgewählt sind: Niederalkoxy oder Nitro;

$R_2$ einen Niederalkylrest oder Phenylrest darstellt, der ggf. durch eine oder mehrere gleiche oder verschiedene Halogengruppen substituiert ist;

$R_{3a}$ und $R_{3b}$ ein Wasserstoffatom darstellen;

$R'_4$ und $R''_4$ unabhängig voneinander ein Wasserstoffatom, einen Nieralkyl- oder Arylrest darstellen;

A---B -C-N($R_5$)- darstellt;

$R_5$ ein,Wasserstoffatom, einen Niederalkenylrest oder einen

Rest der Formel -C(O)-($CH_2$)$_p$-$R'_5$ darstellt.

**6.** Verbindungen der in Anspruch 5 definierten allgemeinen Formel I, in der

$R'_1$ einen Phenylrest darstellt, der ggf. durch einen oder mehrere gleiche oder verschiedene Substituenten substituiert ist, die aus den folgenden Resten ausgewählt sind: Niederalkoxy oder Nitro;

$R_2$ einen Niederalkylrest oder Phenylrest darstellt, der ggf. durch eine Chlorgruppe substituiert ist;

$R'_4$ und $R''_4$ unabhängig voneinander ein Wasserstoffatom, Niederalkyl oder Phenyl darstellen;

$R_5$ ein Wasserstoffatom, Pentenyl oder einen Rest der Formel -C(O)-($CH_2$)$_p$-$R'_5$ darstellt;

$R'_5$ ein Wasserstoffatom, einen der Reste Amino, Cyclopentyl, Indolyl, der. Formel -NH-C(O)-$CH_2$-NH-C(O) -$CH_2$-$NH_2$ oder Phenyl darstellt, der ggf. durch einen oder mehrere gleiche oder verschiedene Substituenten substituiert ist, die aus den Resten Alkyl und Alkoxyalkyl ausgewählt sind, wobei die Reste Alkyl und Alkoxyakyl ihrerseits durch die Reste Oxy und Amino substituiert sind.

**7.** Verbindungen nach einem der Ansprüche 1, 5 oder 6, in denen A---B -C-N($R_5$)- darstellt und die Reste $R_1$; $R'_1$; Y; $R_2$; $R_{3a}$; $R_{3b}$; X; $R_5$; n; $R'_4$; $R''_4$ jeweils die folgenden Bedeutungen haben:

- H; -; -; 2-Cl-Phe; H; H; H; O; 1; H; Phe;
- H; -; -; 2-Cl-Phe; H; H; H; O; 2; Me; Me;
- H; -; -; 2-Cl-Phe; H; H; -$CH_2CH=C(Me)_2$; O; 1; H; Phe;
- H; -; -; 2-Cl-Phe; H; H; Aminohexylcarbonyl; O; 1; H; Phe;
- H; -; -; 2-Cl-Phe; H; H; Aminopentylcarbonyl; O; 1; H; Phe;
- H; -; -; 2-Cl-Phe; H; H; Indolylmethylcarbonyl; O; 1; H; Phe;
- H; -; -; 2-Cl-Phe; H; H; Aminobutylcarbonyl; O; 1; H; Phe;
- H; -; -; 2-Cl-Phe; H; H; Propylcarbonyl; O; 1; H; Phe;
- H; -; -; 2-Cl-Phe; H; H; Cyclopentylmethylcarbonyl; O; 1; H; Phe;
- H; -; -; 2-Cl-Phe; H; H; Phenylpropylcarbonyl; O; 1; H; Phe;
- H; -; -; 2-Cl-Phe; H; H; Phenylethylcarbonyl; O; 1; H; Phe;
- H; -; -; 2-Cl-Phe; H; H; 4-(L-Alanoyloxymethyl)benzylcarbonyl; O; 1; H; Phe;
- H; -; -; 2-Cl-Phe; H; H; 4-(Aminomethyl)phenylcarbonyl; O ; 1 ; H; Phe;
- H; -; -; Phe; H; H; $NH_2$-$CH_2$-C(O)-NH-$CH_2$-C(O)-NH-$CH_2$-C(O)-; O; 2; Me; Me;
- H; -; -; Neopentyl; H; H; Aminohexylcarbonyl; O; 1; H; Phe;
- H; -; -; Isobutyl; H; H; Aminohexylcarbonyl; O; 1; H; Phe;
- H; -; -; Isobutyl; H; H; H; O; 1; H; Phe;
- $R'_1$-NH-C(Y)-; 2-$NO_2$-4-MeO-Phe; S; 2-Cl-Phe; H; H; H; O; 4; H; H;
- $R'_1$-NH-C(Y)-; 2-$NO_2$-4-MeO-Phe; S; 2-Cl-Phe; H; H; -$CH_2CH=C(Me)_2$; O; 1; H; Phe;
- $R'_1$-NH-C(Y)-; 2-$NO_2$-4-MeO-Phe; S; 2-Cl-Phe; H; H; Aminohexylcarbonyl; O; 1; H; Phe;
- $R'_1$-NH-C(Y)-; 2-$NO_2$-4-MeO-Phe; S; 2-Cl-Phe; H; H; Propylcarbonyl; O; 1; H; Phe;
- $R'_1$-NH-C(Y)-; 2-$NO_2$-4-MeO-Phe; S; 2-Cl-Phe; H; H; Cyclopentylmethylcarbonyl; O; 1; H; Phe;
- $R'_1$-NH-C(Y)-; 2-$NO_2$-4-MeO-Phe; S; 2-Cl-Phe; H; H; Phenylpropylcarbonyl; O; 1; H; Phe;
- $R'_1$-NH-C(Y)-; 2-$NO_2$-4-MeO-Phe; S; 2-Cl-Phe; H; H; Phenylethylcarbonyl; O; 1; H; Phe;
- $R'_1$-NH-C(Y)-; 2-$NO_2$-4-MeO-Phe; S; 2-Cl-Phe; H; H; Aminobutylcarbonyl; O; 1; H; Phe;
- $R'_1$-NH-C(Y)-; 2-$NO_2$-4-MeO-Phe; S; 2-Cl-Phe; H; H; Indolylmethylcarbonyl; O; 1; H; Phe;
- $R'_1$-NH-C(Y)-; 2-$NO_2$-4-MeO-Phe; S; 2-Cl-Phe; H; H; Aminopentylcarbonyl; O; 1; H; Phe;
- $R'_1$-NH-C(Y)-; 2-$NO_2$-4-MeO-Phe; S; Phe; H; H; $NH_2$-$CH_2$-C(O)-NH-$CH_2$-C(O)-NH-$CH_2$-C(O)-; O; 2; Me; Me;
- $R'_1$-NH-C(Y)-; 2-$NO_2$-4-MeO-Phe; S; Phe; H; H; Aminohexylcarbonyl; O; 2; Me; Me;

- $R'_1$-NH-C(Y)-; 2-$NO_2$-4-MeO-Phe; S; Neopentyl; H; H; Aminohexylcarbonyl; O; 1; H; Phe;
- $R'_1$-NH-C(Y)-; 2-$NO_2$-4-MeO-Phe; S; Isobutyl; H; H; Aminohexylcarbonyl; O; 1; H; Phe.

**8.** Verfahren zur Herstellung von Verbindungen der in Anspruch 1 definierten allgemeinen Formel I, in der A---B C=N darstellt, $R_1$ ein Wasserstoffatom und $R_{3a}$ ein Wasserstoffatom oder einen Alkylrest darstellt, wobei das Verfahren darin besteht, daß man eine Verbindung der Formel (1)

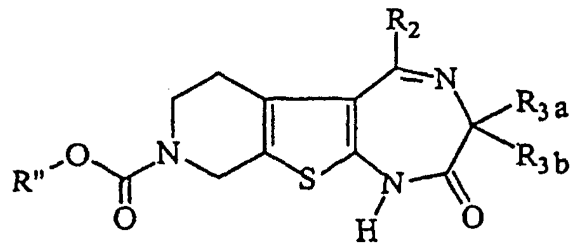

(1)

in der $R_2$ und $R_{3b}$ die in Anspruch 1 angegebene Bedeutung haben, $R_{3a}$ die oben angegebene Bedeutung hat und R'' einen Niederalkyl- oder Arylniederalkylrest darstellt, in Gegenwart einer starken Base mit einer Verbindung $R_4Z$, in der $R_4$ die in Anspruch 1 angegebene Bedeutung hat und Z eine Abgangsgruppe darstellt, reagieren läßt, um die Verbindung (2) zu erhalten

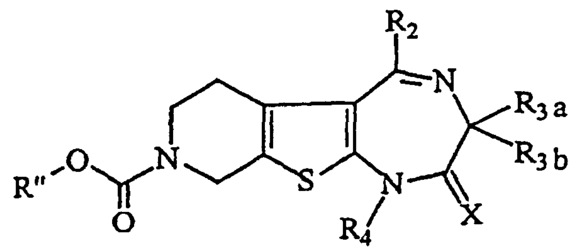

(2)

in der X ein Sauerstoffatom darstellt, wobei man die auf diese Weise erhaltene Verbindung (2) mit einem Thiationsreagenz reagieren lassen kann, um die entsprechende Verbindung zu erhalten, in der X ein Schwefelatom darstellt,
und die Verbindung (2), in der X ein Sauerstoff- oder Schwefelatom darstellt, schließlich einer Reaktion zur Entschützung des Carbamats unterzieht, um das gewünschte Produkt (I) zu erhalten.

**9.** Verfahren zur Herstellung von Verbindungen der in Anspruch 1 definierten allgemeinen Formel I, in der A---B -C=N- darstellt, $R_1$ ein Wasserstoffatom und $R_{3a}$ einen Hydroxyrest darstellt, wobei das Verfahren darin besteht, daß man eine in Anspruch 8 definierte Verbindung der Formel (2) in einem neutralen Lösungsmittel oxidiert, um eine Verbindung der Formel (3) zu erhalten

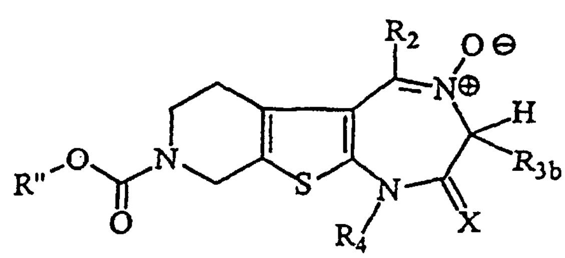

(3)

in der $R_2$, $R_{3b}$, $R_4$ und X die in Anspruch 1 angegebene Bedeutung haben und R" die in Anspruch 8 angegebene Bedeutung hat,
wobei man die Verbindung der Formel (3) mit Essigsäureanhydrid behandelt, um eine Verbindung der Formel (4) zu erhalten

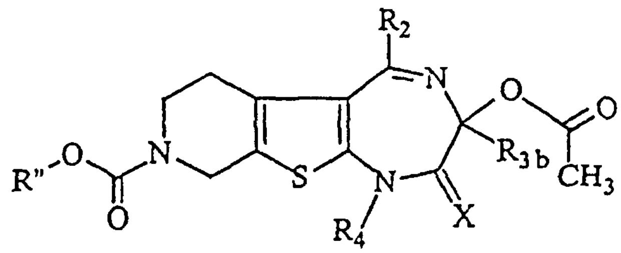

(4)

in der $R_2$, $R_{3b}$, $R_4$, R" und X die oben angegebene Bedeutung haben,
die Verbindung (4) anschließend verseift, um die Verbindung der Formel (5) zu erhalten

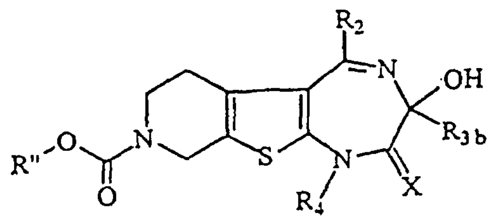

(5)

in der $R_2$, $R_{3b}$, $R_4$, R" und X die oben angegebene Bedeutung haben,
und die Verbindung (5) anschließend einer Reaktion zur Entschützung des Carbamats unterzieht, um die entsprechende Verbindung der Formel (I) zu erhalten, in der $R_1$ H und $R_{3a}$ einen Hydroxyrest darstellt.

**10.** Verfahren zur Herstellung von Verbindungen der in Anspruch 1 definierten allgemeinen Formel I, in der A---B -C=N-, $R_1$ ein Wasserstoffatom und $R_{3a}$ einen Rest -OC(O)-$R'_{3a}$ darstellt, wobei dieses Verfahren darin besteht, daß man eine Verbindung der in Anspruch 9 definierten Formel (5) mit einer Säure der Formel $R'_{3a}$ C(O)OH reagieren läßt, in der $R'_{3a}$ die in Anspruch 1 angegebene Bedeutung hat, um eine Verbindung der Formel (6) zu erhalten

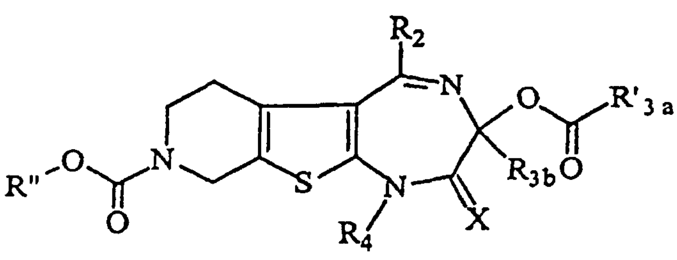

(6)

in der $R_2$, $R'_{3a}$, $R_{3b}$, $R_4$, R" und X die oben angegebene Bedeutung haben,
und diese Verbindung (6) anschließend einer Reaktion zur Entschützung des Carbamats unterzieht, um die entsprechende Verbindung der Formel (I) zu erhalten, in der $R_1$ H und $R_{3a}$ den Rest -OC(O)-$R'_{3a}$ darstellt.

**11.** Verfahren zur Herstellung von Verbindungen der in Anspruch 1 definierten allgemeinen Formel I, in der A---B -C-N (R5)-, $R_1$ und $R_5$ ein Wasserstoffatom und $R_{3a}$ ein Wasserstoffatom oder einen Alkylrest darstellen,
wobei dieses Verfahren darin besteht, daß man ein schwaches Reduktionsmittel in saurem Medium mit einer Verbindung der in Anspruch 8 definierten Formel (2) reagieren läßt, um die Verbindung der Formel (8) zu erhalten,

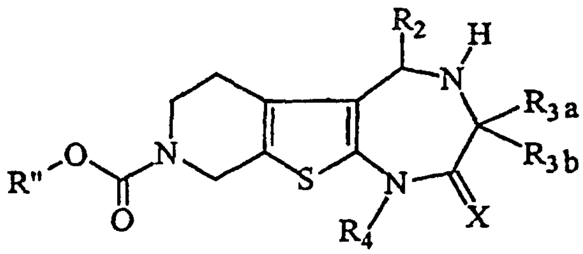

(8)

in der $R_2$, $R_{3b}$, $R_4$, und X die in Anspruch 1 angegebene Bedeutung haben, $R_{3a}$ die oben angegebene Bedeutung hat und R'' die in Anspruch 8 angegebene Bedeutung hat,
und die Verbindung (8) einer Reaktion zur Entschützung des Carbamats unterzieht, um das gewünschte Produkt (I) zu erhalten, in dem $R_1$ ein Wasserstoffatom darstellt.

**12.** Verfahren zur Herstellung von Verbindungen der in Anspruch 1 definierten allgemeinen Formel I, in der A---B -C-N ($R_5$)- darstellt, $R_1$ ein Wasserstoffatom, $R_{3a}$ ein Wasserstoffatom oder einen Alkylrest darstellt und $R_5$ einen Alkenylrest darstellt,
wobei das Verfahren darin besteht, daß man eine Verbindung der in Anspruch 11 definierten Formel (8) mit einer Verbindung der Formel Z'-$R_5$, in der $R_5$ die oben angegebene Bedeutung hat und Z' eine Abgangsgruppe ist, in Gegenwart einer starken mineralischen Base in einem neutralen Lösungsmittel reagieren läßt, um die Verbindung der Formel (9) zu erhalten

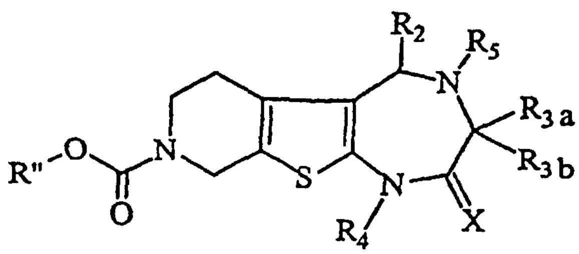

(9)

in der $R_2$, $R_{3b}$, $R_4$ und X die in Anspruch 1 angegebene Bedeutung haben, $R_{3a}$ und $R_5$ die oben angegebene Bedeutung haben und R'' die in Anspruch 8 angegebene Bedeutung hat,
und die Verbindung (9) einer Reaktion zur Entschützung des Carbamats unterzieht, um das gewünschte Produkt (I) zu erhalten, in dem $R_1$ ein Wasserstoffatom darstellt

**13.** Verfahren zur Herstellung von Verbindungen der in Anspruch 1 definierten allgemeinen Formel I, in der A---B -C-N ($R_5$)- darstellt, $R_1$ ein Wasserstoffatom, $R_{3a}$ ein Wasserstoffatom oder einen Alkylrest darstellt und $R_5$ einen Rest $-C-(CH_2)_p-(O)R'_5$ darstellt,
wobei das Verfahren darin besteht, daß man eine Verbindung der in Anspruch 11 definierten Formel (8) mit einer Säure der Formel $R'_5-(CH_2)_p-C(O)OH$, in der $R'_5$ und p die oben angegebene Bedeutung haben, reagieren läßt, um die, entsprechende Verbindung (10) zu erhalten,

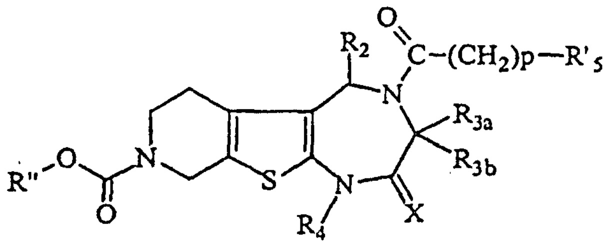

(10)

in der $R_2$, $R_{3b}$, $R_4$, p und X die in Anspruch 1 angegebene Bedeutung haben, $R_{3a}$ und $R'_5$ die oben angegebene Bedeutung haben und R" die in Anspruch 8 angegebene Bedeutung hat,
und die Verbindung (10) einer Reaktion zur Entschützung des Carbamats unterzieht, um das gewünschte Produkt (I) zu erhalten, in dem $R_1$ ein Wasserstoffatom darstellt.

**14.** Verfahren zur Herstellung von Verbindungen der in Anspruch 1 definierten allgemeinen Formel I, in der $R_1$ einen Rest der Formel $R'_1$-NH-C(Y)- darstellt, wobei das Verfahren darin besteht, daß man die entsprechende Verbindung der Formel (I), in der $R_1$ ein Wasserstoffatom darstellt, mit einer Verbindung der Formel

$$R'_1\text{-N=C=Y} \qquad (7)$$

in der $R'_1$ und Y die oben angegebene Bedeutung haben, reagieren läßt, um die gewählte Verbindung der Formel I zu erhalten.

**15.** Verbindungen der in Anspruch 1 definierten Formel I sowie die Additionssalze dieser Produkte der Formel I mit pharmazeutisch verträglichen Mineralsäuren oder organischen Säuren in Form von Medikamenten.

**16.** Verbindungen der in einem der Ansprüche 2 bis 7 definierten Formel I sowie die Additionssalze dieser Produkte der Formel I mit pharmazeutisch verträglichen Mineralsäuren oder organischen Säuren in Form von Medikamenten.

**17.** Pharmazeutische Zusammensetzungen, die als Wirkstoff mindestens eines der in einem der Ansprüche 15 oder 16 definierten Medikamente enthält, in Kombination mit einem pharmazeutisch verträglichen Träger.

**18.** Verwendung einer Verbindung nach einem der Ansprüche 1 bis 7 für die Herstellung eines Medikaments für die Behandlung der Akromegalie, von Hypophyseadenomen und von endokrinen Tumoren des gastroentero-pankreatischen Systems.

**19.** Verbindungen der Formel (2), wie sie in Anspruch 8 definiert sind.

## Claims

**1.** Compounds of general formula I

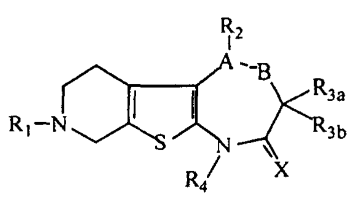

I

under racemic, or enantiomeric or diastereoisomeric form or mixture thereof, and in which

$R_1$ represents the hydrogen atom or a radical of formula $R'_1$-NH-C(Y)- ;

$R'_1$ represents an aryl or heteroaryl radical, the aryl and heteroaryl radicals being optionally substituted by one or more substituents, identical or different, chosen from the following radicals : lower alkyl, lower alkoxy, lower alkylthio, lower alkoxy carbonyl, lower alkyl sulphonyl, halo, trifluoromethyl, trifluoromethyloxy, hydroxy, nitro, cyano, aryl, aryloxy, cycloalkyl or heterocycloalkyl ;

$R_2$ represents a lower alkyl, trifluoromethyl radical or the phenyl radical optionally substituted by one or more substituents, identical or different, chosen from : the hydroxy, halo radical, a lower alkyl or lower alkoxy radical ;

X and Y represent independently O or S ;

$R_{3a}$ represents the hydrogen atom, a lower alkyl, hydroxy radical or the radical of formula -OC(O) $R'_{3a}$ ;

$R'_{3a}$ represents an alkyl radical containing 1 to 10 carbon atoms optionally substituted by one or more substituents, identical or different, chosen from : cycloalkyl ; heterocycloalkyl ; aryl ; heteroaryl ; guanidyl optionally substituted by nitro or cyano ; a radical of formula $NR''_{3a}R'''_{3a}$ in which $NR''_{3a}$ and $R'''_{3a}$ represent, independently, the hydrogen atom, a lower alkyl, aryl, lower arylalkyl, lower heteroarylalkyl, alkylcarbonyl or alkoxycarbonyl radical;

$R_{3b}$ represents the hydrogen atom or a lower alkyl radical ;

$R_4$ represents a radical of formula $-(CH_2)_n-CHR'_4R''_4$ ;

n represents the values 0, 1, 2, 3, 4, 5 or 6 ;

$R'_4$ and $R''_4$ represent, independently, the hydrogen atom, a lower alkyl, cycloalkyl, lower cycloalkyl alkyl, aryl, lower arylalkyl, heteroaryl, lower heteroarylalkyl, arylcarbonyl or adamantyl radical, these radicals being optionally substituted by one or more substituents, identical or different, arylalkyl, heteroaryl, heteroarylalkyl, arylcarbonyl or adamantyl radical chosen from : the hydroxy, halo, trifluoromethyl radical, a lower alkyl or lower alkoxy radical;

A---B represents -C=N- or $-C-N(R_5)-$ ;

$R_5$ represents the hydrogen atom, a lower alkyl, lower alkenyl radical or a radical of formula $-C(O)-(CH_2)_p-R'_5$ ;

$R'_5$ represents the hydrogen atom, the radical amino, lower alkyl amino, di(lower alkyl)amino, cycloalkyl, heterocycloalkyl, guanidyl optionally susbtituted by nitro or cyano ; aryl optionally substituted by one or more substituents, identical or different, chosen from alkyl or alkoxyalkyl radical, these radicals alkyl and alkoxyalkyl being optionally substituted by oxy and amino ; heteroaryl or a radical of formula $-NH-C(O)-(CH_2)_c-NH-C(O)-(CH_2)_d-NH_2$ ;

p represents the values 0, 1, 2, 3, 4, 5, 6, 7, 8, 9 or 10 ;

c and d represent independently the values 0, 1, 2 or 3 ;

or a salt of these compounds ; it being understood that the term « lower » referring to alkyl, alkoxy or alkylthio radical, means a radical containing at least 6 carbon atoms.

**2.** Compounds of general formula I as defined in claim 1 and in which

$R'_1$ represents an aryl radical optionally substituted by one or more substituents, identical or different, chosen from the following radicals : lower alkoxy, trifluoromethyl or nitro;

$R_2$ represents a lower alkyl radical or the radical phenyl optionally substituted by one or more groups, identical or different, chosen from halo or lower alkyl ;

$R_{3a}$ represents the hydrogen atom, hydroxy or the radical of formula -OC(O)$R'_{3a}$ ;

$R'_{3a}$ represents a linear or branched alkyl containing 1 to 6 carbon atoms optionally substituted by one or more substituents, identical or different, of formula $NR''_{3a}R'''_{3a}$ in which R"3a and $R'''_{3a}$ represent, independently, the hydrogen atom, a lower alkyl or alkoxycarbonyl;

$R_{3b}$ represents the hydrogen atom ;

$R'_4$ and $R''_4$ represent, independently, the hydrogen atom, a lower alkyl, cycloalkyl, aryl, heteroaryl, arylcarbonyl or adamantyl radical ;

A---B represents -C=N.

**3.** Compounds of general formula I as defined in claim 2 and in which

$R'_1$ represents a phenyl radical optionally substituted by one or more substituents, identical or different, chosen from the following radicals : lower alkoxy, trifluoromethyl or nitro;

$R_2$ represents a lower alkyl radical or the phenyl radical optionally substituted by one or more groups, identical or different, chosen from : lower alkyl, chloro or fluoro ;

$R'_{3a}$ represents a linear or branched alkyl containing 1 to 6 carbon atoms optionally substituted by one or more amino groups ;

$R'_4$ and $R''_4$ represent, independently, the hydrogen atom, lower alkyl, cyclohexyl, phenyl, pyridyl, phenylcarbonyl or adamantyl.

**4.** Compounds according to one of claims 1 to 3 in which A---B represents -C=N and the $R_1$, $R'_1$, Y, $R_2$, $R_{3a}$, $R_{3b}$, X, n, $R'_4$, $R''_4$ radicals respectively have the following meanings :

H ; - ; - ; 2-Cl-Phe ; H ; H ; O ; 1 ; H ; Phe ;

H ; - ; - ; 2-Cl-Phe ; H ; H ; S ; 1 ; H ; Phe ;

H ; - ; - ; 2-Cl-Phe ; H ; H ; O ; 2 ; H ; Phe ;

H; - ; - ; 2-Cl-Phe ; H ; H ; O ; 0 ; H ; phenylcarbonyl ;

H ; - ; - ; 2-Cl-Phe ; H ; H ; O ; 0 ; H ; Phe ;

H ; - ; - ; 2-Cl-Phe ; H ; H ; O ; 0 ; H ; cyclohexyl ;

H ; - ; - ; 2-Cl-Phe ; H ; H ; O ; 4 ; H ; H ;

H ; - ; - ; 2-Cl-Phe ; H ; H ; O ; 2 ; Phe ; Phe ;

H ; - ; - ; 2-Cl-Phe ; H ; H ; O ; 2 ; Me ; Me ;

H ; - ; - ; 2-Cl-Phe ; H ; H ; O ; 0 ; H ; adamantyl ;

H ; - ; - ; 2-Cl-Phe ; H ; H ; O ; 1 ; H ; pyridyl ;

H ; - ; - ; Phe ; H ; H ; O ; 1 ; H ; Phe ;

H ; - ; - ; 4-Cl-Phe ; H ; H ; O ; 1 ; H ; Phe ;

H ; - ; - ; 2-F-Phe ; H ; H ; O ; 1 ; H ; Phe ;

H ; - ; - ; 4-F-Phe ; H ; H ; O ; 1 ; H ; Phe ;

H ; - ; - ; 2-Me-Phe ; H ; H ; O ; 1 ; H ; Phe ;

H ; - ; - ; t-butyl ; H ; H ; O ; 1H ; Phe ;

H ; - ; - ; 2-Cl-Phe ; OH ; H ; O ; 1 ; H ; Phe ;

H ; - ; - ; 2-Cl-Phe ; OC(O)-$(CH_2)_6NH_2$ ; H ; O ; 1 ; H ; Phe ;

R'$_1$-NH-C(Y)- ; 2-$NO_2$-4-MeO-Phe ; S ; 2-Cl-Phe ; H ; H ; O ; 1 ; H ; Phe ;

R'$_1$-NH-C(Y)- ; 2-$NO_2$-4-MeO-Phe ; S ; 2-Cl-Phe ; H ; H ; O ; 2 ; H ; Phe ;

R'$_1$-NH-C(Y)- ; 2-$NO_2$-4-MeO-Phe ; S ; 2-Cl-Phe ; H ; H ; S ; 1 ; H ; Phe ;

R'$_1$-NH-C(Y)- ; 2-$NO_2$-4-MeO-Phe ; S ; 2-Cl-Phe ; H ; H ; O ; 0 ; H ; phenylcarbonyl;

R'$_1$-NH-C(Y)- ; 2-$NO_2$-4-MeO-Phe ; S ; 2-Cl-Phe ; H ; H ; O ; 0 ; H ; Phe ;

R'$_1$-NH-C(Y)- ; 2-$NO_2$-4-MeO-Phe ; S ; 2-Cl-Phe ; H ; H ; O ; 4 ; H ; H ;

R'$_1$-NH-C(Y)- ; 2-$NO_2$-4-MeO-Phe ; S ; 2-Cl-Phe ; H ; H ; O ; 0 ; H ; cyclohexyl;

R'$_1$-NH-C(Y)- ; 2-$NO_2$-4-MeO-Phe ; S ; 2-Cl-Phe ; H ; H ; O ; 2 ; Phe ; Phe ;

R'$_1$-NH-C(Y)- ; 2-$NO_2$-4-MeO-Phe ; S ; 2-Cl-Phe ; H ; H ; O ; 2 ; Me ; Me ;

R'$_1$-NH-C(Y)- ; 2-$NO_2$-4-MeO-Phe ; S ; 2-Cl-Phe ; H ; H ; O ; 0 ; H ; adamantyl;

R'$_1$-NH-C(Y)- ; 2-$NO_2$-4-MeO-Phe ; S ; 2-Cl-Phe ; H ; H ; O ; 1 ; H ; pyridyl;

R'$_1$-NH-C(Y)- ; 2-$NO_2$-4-MeO-Phe ; S ; Phe ; H ; H ; O ; 1 ; H ; Phe ;

R'$_1$-NH-C(Y)- ; 2-$NO_2$-4-MeO-Phe ; S ; 4-Cl-Phe ; H ; H ; O ; 1 ; H ; Phe ;

R'$_1$-NH-C(Y)- ; 2-$NO_2$-4-MeO-Phe ; S ; 2-F-Phe ; H ; H ; O ; 1 ; H ; Phe ;

R'$_1$-NH-C(Y)- ; 2-$NO_2$-4-MeO-Phe ; S ; 4-F-Phe ; H ; H ; O ; 1 ; H ; Phe ;

R'$_1$-NH-C(Y)- ; 2-$F_3C$-Phe ; O ; 4-F-Phe ; H ; H ; O ; 1 ; H ; Phe ;

R'$_1$-NH-C(Y)- ; 2-$NO_2$-4-MeO-Phe ; S ; 2-Me-Phe ; H ; H ; O ; 1 ; H ; Phe ;

R'$_1$-NH-C(Y)- ; 2-$NO_2$-4-MeO-Phe ; S ; t-butyl ; H ; H ; O ; 1 ; H ; Phe ;

R'$_1$-NH-C(Y)- ; 2-$NO_2$-4-MeO-Phe ; S ; 2-Cl-Phe ; OH ; H ; O ; 1 ; H ; Phe ;

R'$_1$-NH-C(Y)- ; 2-$NO_2$-4-MeO-Phe ; S ; 2-Cl-Phe ; OC(O)-$(CH_2)_6NH_2$ ; H ; O ; 1 ; H ; Phe ;

R'$_1$-NH-C(Y)- ; 2-$NO_2$-4-MeO-Phe ; S ; 2-Me-Phe ; H ; H ;O ; 1 ; H ; Phe ;

R'$_1$-NH-C(Y)- ; 2-$NO_2$-4-MeO-Phe ; S ; t-butyle ; H ; H ; O ; 1 ; H ; Phe ;

R'$_1$-NH-C(Y)- ; 2-$NO_2$-4-MeO-Phe ; S ; 2-Cl-Phe ; OH ; H ; O ; 1 ; H ; Phe ;

R'$_1$-NH-C(Y)- ; 2-$NO_2$-4-MeO-Phe ; S ; 2-Cl-Phe ; OC(O)-$(CH_2)_6NH_2$ ; H ; O ; 1 ; H ; Phe.

**5.** Compounds of general formula I as defined in claim 1 and in which

$R'_1$ represents an aryl radical optionally substituted by one or more substituents, identical or different, chosen from the following radicals : lower alkoxy or nitro ;

$R_2$ represents a lower alkyl or a phenyl radical optionally substituted by one or more identical or different halo groups ;

$R_{3a}$ and $R_{3b}$ represent the hydrogen atom ;

$R'_4$ and $R''_4$ represent, independently, the hydrogen atom, a lower alkyl or aryl radical ;

A---B represents -C-N($R_5$)- ;

$R_5$ represents the hydrogen atom, a lower alkenyl radical or a radical of formula -C(O)-($CH_2$)$_p$-$R'_5$.

**6.** Compounds of general formula I as defined in claim 5 and in which

$R'_1$ represents a phenyl radical optionally substituted by one or more substituents, identical or different, chosen from the following radicals : lower alkoxy or nitro ;

$R_2$ represents a lower alkyl or phenyl radical optionally substituted by a chloro group;

$R'_4$ and $R''_4$ represent, independently, the hydrogen atom, lower alkyl or phenyl;

$R_5$ represents the hydrogen atom, pentenyl or a radical of formula -C(O)-($CH_2$)$_p$-$R'_5$ ;

$R'_5$ represents the hydrogen atom, amino, cyclopentyle, indolyle, a radical of formula -NH-C(O)-$CH_2$-NH-C(O) -$CH_2$-$NH_2$, or phenyl optionally substituted by one or more substituents, identical or different, chosen from alkyl and alkoxyalkyl radicals, these radicals alkyl and alkoxyalkyl being optionally substituted by oxy and amino.

**7.** Compounds according to one of claims 1, 5 or 6 in which A---B represents -C-N($R_5$)-and the $R_1$ ; $R'_1$ ; Y ; $R_2$ ; $R_{3a}$ ; $R_{3b}$ ; X ; $R_5$ ; n ; $R'_4$ ; $R''_4$ radicals have respectively the following meanings :

H; -; -; 2-Cl-Phe; H; H; H; O; 1; H; Phe;

H; -; -; 2-Cl-Phe; H; H; H; O; 2; Me; Me;

H; -; -; 2-Cl-Phe; H; H; -$CH_2CH=C(Me)_2$; O; 1; H; Phe;

H; -; -; 2-Cl-Phe; H; H; aminohexylcarbonyl; O; 1; H; Phe;

H; -; -; 2-Cl-Phe; H; H; aminopentylcarbonyl; O; 1; H; Phe;

H; -; -; 2-Cl-Phe; H; H; indolylmethylcarbonyl; O; 1; H; Phe;

H; -; -; 2-Cl-Phe; H; H; aminobutylcarbonyl; O; 1; H; Phe;

H; -; -; 2-Cl-Phe; H; H; propylcarbonyl; O; 1; H; Phe;

H; -; -; 2-Cl-Phe; H; H; cyclopentyl-methylcarbonyl; O; 1; H; Phe;

H; -; -; 2-Cl-Phe; H; H; phenyl-propylcarbonyl; O; 1; H; Phe;

H; -; -; 2-Cl-Phe; H; H; phenylethylcarbonyl; O; 1; H; Phe;

H ; - ; - ; 2-Cl-Phe ; H ; H ; 4-(L-alanoyloxymethyl)benzyl carbonyl ; O ; 1 ; H ; Phe ;

H ; - ; - ; 2-Cl-Phe ; H ; H ; 4-(aminomethyl)phenyl carbonyl ; O ; 1 ; H ; Phe ;

H ; - ; - ; Phe ; H ; H ; $NH_2$-$CH_2$-C(O)-NH-$CH_2$C(O)-NH-$CH_2$-C(O)-; O ; 2 ; Me ; Me ;

H ; - ; - ; neopentyl ; H ; H ; aminohexylcarbonyl ; O ; 1 ; H ; Phe ;

H ; - ; - ; isobutyl ; H ; H ; aminohexylcarbonyl ; O ; 1 ; H ; Phe ;

H ; - ; - ; isobutyl ; H ; H ; H; O ; 1 ; H ; Phe ;

R'$_1$-NH-C(Y)-; 2-$NO_2$-4-MeO-Phe; S; 2-Cl-Phe; H; H; H; O; 4; H; H;

R'$_1$-NH-C(Y)-; 2-$NO_2$-4-MeO-Phe; S; 2-Cl-Phe; H; H; -$CH_2$CH=C$(Me)_2$; 0; 1; H; Phe;

R'$_1$-NH-C(Y)-; 2-$NO_2$-4-MeO-Phe; S; 2-Cl-Phe; H; H; aminohexylcarbonyl; O; 1; H; Phe;

R'$_1$-NH-C(Y)-; 2-$NO_2$-4-MeO-Phe; S; 2-Cl-Phe; H; H; propylcarbonyl; O; 1; H; Phe;

R'$_1$-NH-C(Y)-; 2-$NO_2$-4-MeO-Phe; S; 2-Cl-Phe; H; H; cyclopentyl-methylcarbonyl; O; 1; H; Phe;

R'$_1$-NH-C(Y)-; 2-$NO_2$-4-MeO-Phe; S; 2-Cl-Phe; H; H; phenyl-propylcarbonyl; O; 1; H; Phe;

R'$_1$-NH-C(Y)-; 2-$NO_2$-4-MeO-Phe; S; 2-Cl-Phe; H; H; phenylethylcarbonyl; O; 1; H; Phe;

R'$_1$-NH-C(Y)-; 2-$NO_2$-4-MeO-Phe; S; 2-Cl-Phe; H; H; aminobutylcarbonyl; O; 1; H; Phe;

R'$_1$-NH-C(Y)-; 2-$NO_2$-4-MeO-Phe; S; 2-Cl-Phe; H; H; indolylmethylcarbonyl; O; 1; H; Phe;

R'$_1$-NH-C(Y)-; 2-$NO_2$-4-MeO-Phe; S; 2-Cl-Phe; H; H; aminopentylcarbonyl; O; 1; H; Phe;

R'$_1$-NH-C(Y)- ; 2-$NO_2$-4-MeO-Phe ; S ; Phe ; H ; H ; $NH_2$-$CH_2$-C(O)-NH-$CH_2$-C(O)-NH-$CH_2$-C(O)- ; O ; 2 ; Me ; Me ;

R'$_1$-NH-C(Y)- ; 2-$NO_2$-4-MeO-Phe ; S ; Phe ; H ; H ; aminohexylcarbonyl ; O ; 2 ; Me ; Me ;

R'$_1$-NH-C(Y)- ; 2-$NO_2$-4-MeO-Phe ; S ; néopentyl ; H ; H ; aminohexylcarbonyl ; O ; 1 ; H ; Phe ;

R'$_1$-NH-C(Y)- ; 2-$NO_2$-4-MeO-Phe ; S ; isobutyl ; H ; H ; aminohexylcarbonyl ; O ; 1 ; H ; Phe.

**8.** Process for the preparation of compounds of general formula I as defined in claim 1 and in which A---B represents C=N, $R_1$ the hydrogen atom and $R_{3a}$ the hydrogen atom or an alkyl radical, which process consists of reacting a compound of formula (1)

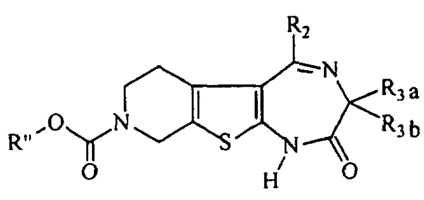

(1)

in which $R_2$, and $R_{3b}$ have the meaning indicated in claim 1, $R_{3a}$ has the meaning indicated above and R" represents a lower alkyl or lower arylalkyl radical, with a compound $R_4Z$ in which $R_4$ has the meaning indicated in claim 1 and Z represents a parting group, in the presence of a strong base, in order to obtain compound (2)

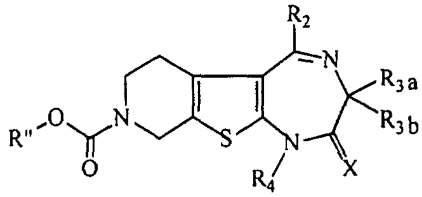

(2)

in which X represents the oxygen atom, compound (2) thus obtained which can be reacted with a thiation reagent in order to obtain compound (2) in which X represents a sulphur atom, compound (2) in which X represents an oxygen or sulphur atom, which is finally subjected to a deprotection reaction of the carbamate in order to obtain the desired product (I).

**9.** Process for the preparation of compounds of general formula I as defined in claim I and in which A---B represents -C=N-, $R_1$ the hydrogen atom and $R_{3a}$ the hydroxy radical, which process consists of oxidizing a compound of formula (2) as defined in claim 9, in an inert solvent in order to obtain a compound of formula (3)

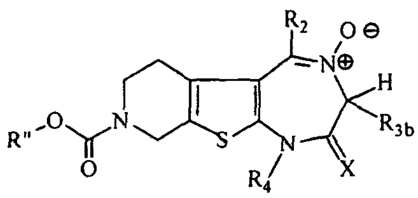

(3)

in which $R_2$, $R_{3b}$, $R_4$ and X have the meaning indicated in claim 1 and R" has the meaning indicated in claim 8, which compound of formula (3) is treated with acetic anhydride in order to obtain a compound of formula (4)

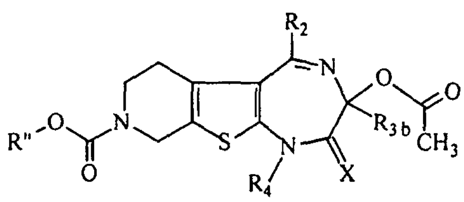

(4)

in which $R_2$, $R_{3b}$, $R_4$, R" and X have the meaning indicated above,
which compound (4) is then saponified in order to obtain the compound of formula (5)

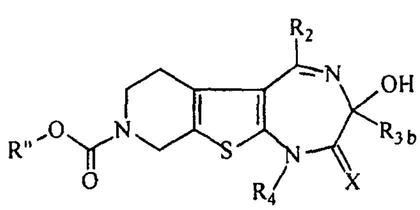

(5)

in which $R_2$, $R_{3b}$, $R_4$, R" and X have the meaning indicated above,
which compound (5) is finally subjected to a deprotection reaction of the carbamate in order to obtain the corresponding compound of formula (I) in which $R_1$ represents H and $R_{3a}$ the hydroxy radical.

**10.** Process for the preparation of compounds of general formula I as defined in claim 1 and in which A---B represents -C=N-, $R_1$ the hydrogen atom and $R_{3a}$ an -OC(O)-$R'_{3a}$ radical, which process consists of reacting a compound of formula (5) as defined in claim 9, with an acid of formula $R'_{3a}C(O)OH$ in which $R'_{3a}$ has the meaning indicated in claim 1, in order to obtain a compound of formula (6)

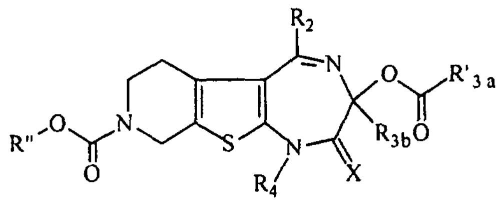

(6)

in which $R_2$, $R'_{3a}$, $R_{3b}$, $R_4$, R" and X have the meaning indicated above,
which compound (6) is finally subjected to a deprotection reaction of the carbamate in order to obtain the corresponding compound of formula (I) in which $R_1$ represents H and $R_{3a}$ the -OC(O)-$R'_{3a}$ radical.

**11.** Process for the preparation of compounds of general formula I as defined in claim 1 and in which A---B represents -C-N($R_5$)-, $R_1$ and $R_5$ a hydrogen atom and $R_{3a}$ the hydrogen atom or an alkyl radical,
which process consists of reacting a gentle reducing agent in acid medium on a compound of formula (2) as defined in claim 8, with, in order to obtain the compound of formula (8)

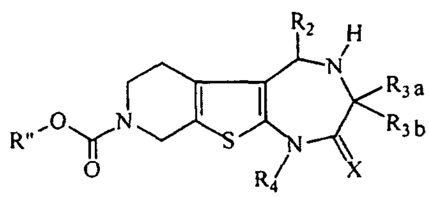

(8)

in which $R_2$, $R_{3b}$, $R_4$ and X have the meaning indicated in claim 1, $R_{3a}$ has the meaning indicated above and R" has the meaning indicated in claim 8,
which compound (8) is subjected to a deprotection reaction of the carbamate in order to obtain the desired product (I) in which $R_1$ represents the hydrogen atom.

**12.** Process for the preparation of compounds of general formula I as defined in claim 1 and in which A---B represents -C-N($R_5$)-, $R_1$ represents a hydrogen atom, $R_{3a}$ the hydrogen atom or an alkyl radical and $R_5$ represents an alkenyl radical,
which process consists of reacting a compound of formula (8) as defined in claim 11, with a compound of formula Z'-$R_5$ in which $R_5$ has the meaning indicated above and Z' a parting group, in the presence of a strong mineral base in an inert solvent, in order to obtain compound (9)

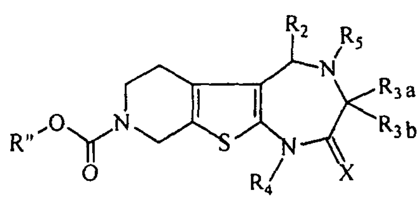

(9)

in which $R_2$, $R_{3b}$, $R_4$ and X have the meaning indicated in claim 1, $R_{3a}$ and $R_5$ have the meaning indicated above and R" has the meaning indicated in claim 8,
which compound (9) is subjected to a deprotection reaction of the carbamate in order to obtain the desired product (I) in which $R_1$ represents the hydrogen atom.

**13.** Process for the preparation of compounds of general formula I as defined in claim 1 and in which A---B represents $-C-N(R_5)-$, $R_1$ represents a hydrogen atom, $R_{3a}$ the hydrogen atom or an alkyl radical and $R_5$ represents a $-C(O)-(CH_2)_p-R'_5$ radical,
which process consists of reacting a compound of formula (8) as defined in claim 11, with an acid of formula $R'_5-(CH_2)_p-C(O)OH$ in which $R'_5$ and p have the meaning indicated above, in order to obtain the corresponding compound (10),

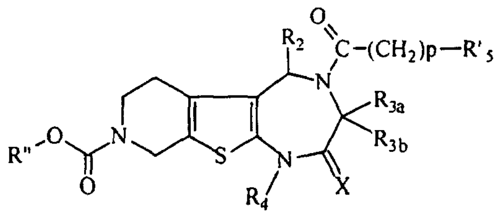

(10)

in which $R_2$, $R_{3b}$, $R_4$ and X have the meaning indicated in claim 1, $R_{3a}$ and $R'_5$ have the meaning indicated above and R" has the meaning indicated in claim 8,
which compound (10) is subjected to a deprotection reaction of the carbamate in order to obtain the desired product (I) in which $R_1$ represents the hydrogen atom.

**14.** Process for the preparation of compounds of general formula I as defined in claim 1 and in which $R_1$ represents a radical of formula $R'_1-NH-C(Y)-$, which process consists of reacting the corresponding compound of formula (I) in which $R_1$ represents the hydrogen atom, with a compound of formula

$$R'_1-N=C=Y \qquad (7)$$

in which $R'_1$ and Y have the meaning indicated above, in order to form the chosen compound of formula I.

**15.** As medicaments, the compounds of formula I as defined in claim 1, as well as the addition salts with pharmaceutically acceptable mineral or organic acids of said products of formula I.

**16.** As medicaments, the compounds of formula I as defined in one of claims 2 to 7, as well as the addition salts with pharmaceutically acceptable mineral or organic acids of said products of formula I.

**17.** Pharmaceutical compositions containing, as active ingredient, at least one of the medicaments as defined in one of claims 15 or 16, in combination with a pharmaceutically acceptable support.

**18.** Use of a compound of formula I as defined in one of claims 1 to 7, for the preparation of a medicament for the treatment of acromegalia, hypophyseal adenomas and endocrinic gastroenteropancreatic tumors.

**19.** Compounds of formula (2) as defined in claim 8.